(19) Europäisches Patentamt
European Patent Office
Office européen des brevets

(11) Publication number: **0 097 039**
**A1**

# (12) EUROPEAN PATENT APPLICATION

(21) Application number: **83303361.6**

(51) Int. Cl.³: **C 07 H 19/08**, A 61 K 31/70

(22) Date of filing: **10.06.83**

(30) Priority: **16.06.82 GB 8217449**
**02.03.83 GB 8305702**

(43) Date of publication of application: **28.12.83**
**Bulletin 83/52**

(84) Designated Contracting States: **CH DE FR GB IT LI NL**

(71) Applicant: **BEECHAM GROUP PLC, Beecham House Great West Road, Brentford Middlesex (GB)**

(72) Inventor: **Harnden, Michael Raymond, 47 Guildford Road, Horsham Sussex (GB)**
Inventor: **Jarvest, Richard Lewis, 29 Beaconsfield Road, Surbiton Surrey (GB)**

(74) Representative: **Russell, Brian John et al, European Patent Attorney Beecham Pharmaceuticals Great Burgh Yew Tree Bottom Road, Epsom Surrey, KT18 5XQ (GB)**

(54) **5-(E-2-halovinyl)-2'-deoxyuridine derivatives, processes for their preparation, pharmaceutical compositions containing them, and their use in treating viral infections.**

(57) Compounds of formula (I):

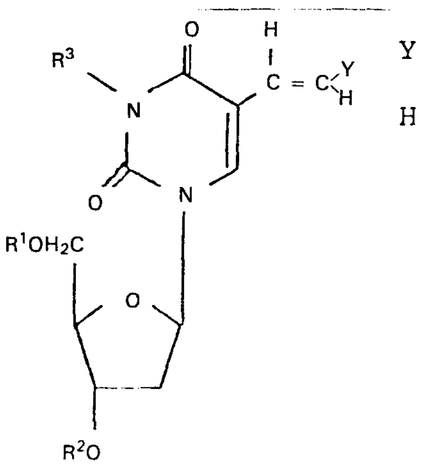

(I)

in which one of $R^1$ or $R^2$ is a group of formula RCH(X)CO.-, and the other is selected from: a hydrogen atom, a tri-$C_{1-6}$ alkylsilyl group, and a group of formula: RCH(X)CO.-
wherein X is a halogen atom, a $C_{1-6}$ alkyloxy group, a phenoxy group, a hydroxyl group, a tri-$C_{1-6}$ alkylsilyloxy group, or a group of formula:

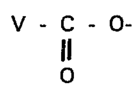

wherein V is a $C_{1-6}$ alkyl group optionally substituted with halophenoxy, or is a phenyl group optionally substituted with halogen, nitro, $C_{1-4}$-alkyl, hydroxy, amino, $C_{1-6}$-alkylamino, N,N-di-$C_{1-6}$ alkylsuphamoyl, or $C_{1-4}$ alkyloxy, and R is a hydrogen atom, a $C_{1-6}$ alkyl group, a phenyl group, a benzyl group, or a phenyl or benzyl group substituted in the aromatic ring with halo, nitro, $C_{1-4}$-alkyl, hydroxy, amino, $C_{1-6}$-alkylamino, or $C_{1-4}$ alkyloxy; $R^3$ is a hydrogen atom, a $C_{1-6}$ alkanoyl group, a benzoyl group or a benzoyl group substituted with halo, nitro, $C_{1-4}$ alkyl, hydroxy, amino, N,N-di-$C_{1-6}$-alkylsulphamoyl, $C_{1-6}$ alkylamino, or $C_{1-4}$ alkyloxy; and Y is a halogen atom, possess antiviral activity, and are useful in the treatment of infections caused by herpes viruses.

EP 0 097 039 A1

# 5-(E-2-HALOVINYL)-2'-DEOXYURIDINE DERIVATIVES, PROCESSES FOR THEIR PREPARATION, PHARMACEUTICAL COMPOSITIONS CONTAINING THEM, AND THEIR USE IN TREATING VIRAL INFECTIONS

This invention relates to certain 2'-deoxyuridine compounds which have antiviral activity.

European Published Patent Specification No. 61283 discloses certain mono- and di-esters of 5-(E-2-bromovinyl)-2'-deoxyuridine which have antiviral activity, particularly against herpes viruses.

We have now found a group of mono-, di- and trisubstituted 5-(2-halogenovinyl)-2'-deoxyuridines which have antiviral activity, and which are useful in the treatment of infections caused by herpes viruses, such as herpes simplex type 1, herpes simplex type 2 and varicella.

According to the present invention there is provided a compound of formula (I):

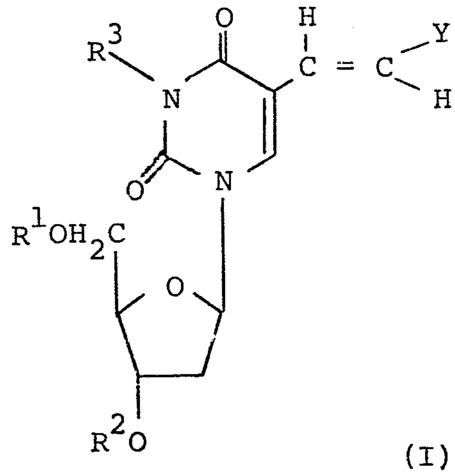

(I)

in which one of $R^1$ or $R^2$ is a group of formula RCH(X)CO.-, and the other is selected from: a hydrogen atom, a tri-$C_{1-6}$ alkylsilyl group, and a group of formula:

RCH(X)CO.-

wherein X is a halogen atom, a $C_{1-6}$ alkyloxy group, a phenoxy group, a hydroxyl group, a tri-$C_{1-6}$ alkylsilyloxy group, or a group of formula:

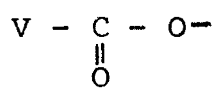

wherein V is a $C_{1-6}$ alkyl group optionally substituted with halophenoxy, or is a phenyl group optionally substituted with halogen, nitro, $C_{1-4}$-alkyl, hydroxy, amino, $C_{1-6}$-alkylamino, N,N-di-$C_{1-6}$ alkylsuphamoyl, or $C_{1-4}$ alkyloxy, and R is a hydrogen atom, a $C_{1-6}$ alkyl group, a phenyl group, a benzyl group, or a phenyl or benzyl group substituted in the aromatic ring with halo, nitro, $C_{1-4}$-alkyl, hydroxy, amino, $C_{1-6}$-alkylamino, or $C_{1-4}$ alkyloxy; $R^3$ is a hydrogen atom, a $C_{1-6}$ alkanoyl group, a benzoyl group or a benzoyl group substituted with halo, nitro, $C_{1-4}$ alkyl,

hydroxy, amino, N,N-di-$C_{1-6}$-alkylsulphamoyl, $C_{1-6}$ alkylamino, or $C_{1-4}$ alkyloxy; and Y is a halogen atom,

Suitably, X is a halogen atom, preferably a chlorine atom, a hydroxyl group, a $C_{1-6}$ alkyloxy group or a group VCO.O-, as defined above. Preferably Y is a bromine atom. Suitably $R^3$ is a hydrogen atom.

Examples of compounds of the invention are as follows:

E-5-(2-bromovinyl)-3',5'-bis(chloroacetyl)-2'-deoxyuridine;
E-5-(2-bromovinyl)-5'-chloroacetyl-2'-deoxyuridine;
E-5-(2-bromovinyl)-3'-chloroacetyl-2'-deoxyuridine;
E-5-(2-bromovinyl)-3',5'-bis(2-chloropropionyl)-2'-deoxyuridine;
E-5-(2-bromovinyl)-5'-(2-chloropropionyl)-2'-deoxyuridine;
E-5-(2-bromovinyl)-3',5'-bis(4-dipropylsulphamoylbenzoyloxyacetyl)-2'-deoxyuridine;
E-5-(2-bromovinyl)-5'-(4-dipropylsulphamoylbenzoyloxyacetyl)-2'-deoxyuridine;
E-5-(2-bromovinyl)-3'-(4-dipropylsulphamoylbenzoyloxyacetyl)-2'-deoxyuridine;
E-5-(2-bromovinyl)-3',5'-bis[2-(4-chlorophenoxy)-2-methylpropionyloxyacetyl]-2'-deoxyuridine;
E-5-(2-bromovinyl)-5'-(2-acetoxypropionyl)-2'-deoxyuridine;
E-5-(2-bromovinyl)-5'-(2-valeryloxypropionyl)-2'-deoxyuridine;
E-5-(2-bromovinyl)-5'-(2-benzoyloxypropionyl)-2'-deoxyuridine;
E-5-(2-bromovinyl)-5'-[2-(4-dipropylsulphamoylbenzoyloxy)propionyl]-2'-deoxyuridine;

E-5-(2-bromovinyl)-3'-trimethylsilyl-5'-(4-dipropylsulphamoylbenzoyloxyacetyl)-2'-deoxyuridine;

3-(4-dipropylsulphamoylbenzoyl)-E-5-(2-bromovinyl)-5'-(4-dipropylsulphamoylbenzoyloxyacetyl)-2'-deoxyuridine;

E-5-(2-bromovinyl)-5'-(L-2-t-butyldimethylsilyloxy-3-phenylpropionyl)-2'-deoxyuridine;

E-5-(2-bromovinyl)-3',5'-bis(L-2-t-butyldimethylsilyloxy-3-phenylpropionyl)-2'-deoxyuridine;

E-5-(2-bromovinyl)-5'-(L-2-hydroxy-3-phenylpropionyl)-2'-deoxyuridine;

E-5-(2-bromovinyl)-3',5'-bis(L-2-hydroxy-3-phenylpropionyl)-2'-deoxyuridine;

E-5-(2-bromovinyl)-3'-(2-chlorophenylacetyl)-2'-deoxyuridine;

E-5-(2-bromovinyl)-5'-(2-chlorophenylacetyl)-2'-deoxyuridine;

E-5-(2-bromovinyl)-3',5'-bis(2-chlorophenylacetyl)-2'-deoxyuridine;

E-5-(2-bromovinyl)-5'-(2-L-hydroxyphenylacetyl)-2'-deoxyuridine;

E-5-(2-bromovinyl)-3',5'-bis(2-L-hydroxyphenylacetyl)-2'-deoxyuridine;

E-5-(2-bromovinyl)-5'-(L-2-hydroxy-4-methylpentanoyl)-2'-deoxyuridine;

E-5-(2-bromovinyl)-3',5'-bis(L-2-hydroxy-4-methylpentanoyl)-2'-deoxyuridine;

E-5-(2-bromovinyl)-5'-methoxyacetyl-2'-deoxyuridine;

E-5-(2-bromovinyl)-3',5'-bis(methoxyacetyl)-2'-deoxyuridine;

E-5-(2-bromovinyl)-5'-(2-D-hydroxyphenylacetyl)-2'-deoxyuridine;

E-5-(2-bromovinyl)-3',5'-bis(2-D-hydroxyphenylacetyl)-2'-deoxyuridine;

E-5-(2-bromovinyl)-5'-(L-2-t-butyldimethylsilyloxy-4-methylpentanoyl)-2'-deoxyuridine;

E-5-(2-bromovinyl)-3',5'-bis(L-2-t-butyldimethylsilyloxy-4-methylpentanoyl)-2'-deoxyuridine;

*E*-5-(2-bromovinyl)-5'-(2-bromobutyryl)-2'-deoxyuridine;
*E*-5-(2-bromovinyl)-3',5'-*bis*(2-bromobutyryl))-2'-deoxyuridine;
*E*-5-(2-bromovinyl)-5'-phenoxyacetyl-2'-deoxyuridine; and
*E*-5-(2-bromovinyl)-3',5'-*bis*(phenoxyacetyl)-2'-deoxyuridine.

Compounds of formula (I) wherein $R^1$, $R^2$ and Y are as defined and $R^3$ is a hydrogen atom may be prepared by treating a compound of formula (II):

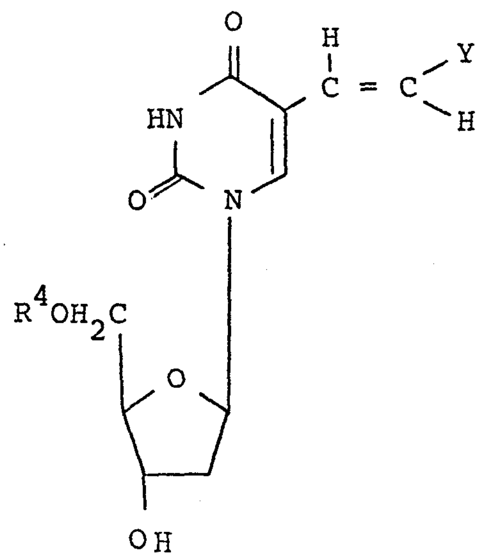

(II)

in which Y is as defined in formula (I), $R^4$ is a hydrogen atom or a tri-$C_{1-6}$-alkylsilyl group, with an acylating agent of formula (III):

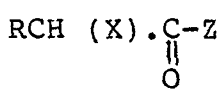

(III)

in which Z is a good leaving group, such as a halogen atom, and R and X are as defined in formula (I), provided X is not a hydroxyl group.

The reaction is suitably carried out in an anhydrous polar solvent, such as anhydrous dioxane, at room temperature in the presence of an amine base, preferably triethylamine. The reaction will generally yield a mixture of products wherein; (i) only one of $R^1$ and $R^2$ is a group RCH.(X).CO.- and (ii) both $R^1$ and $R^2$ are groups of formula RCH.(X).CO.- and the products can be separated by, for example, column chromatography on silica gel.

The acylating agents of formula (III) are either known compounds or can be prepared from known compounds by conventional means.

Compounds of formula (II), wherein $R^4$ is a tri-$C_{1-6}$-alkylsilyl group may be prepared by treating a compound of formula (II), wherein $R^4$ is a hydrogen atom i.e. a 5-(2-halogenovinyl)-2'-deoxyuridine, with a tri-$C_{1-6}$-alkyl silylating agent, such as _t_-butyldimethylsilylchloride or _t_-butyldiphenylsilylchloride in which case the silylation reaction is preferably carried out in an anhydrous polar organic solvent, suitably tetrahydrofuran, at room temperature.

5-(2-Halogenovinyl)-2'-deoxyuridines are disclosed in UK Patent Specification No. 1,601,020.

Compounds of formula (I), in which $R^1$, $R^2$ and Y are as defined, $R^3$ is a hydrogen atom, and X is a hydroxyl group, may be prepared by treating the compound of formula (II), as defined above, with a compound of formula (III), in which X is a protected hydroxyl group and R and Z are as defined, and subsequently deprotecting the product. The reaction is suitably carried out in an anhydrous polar organic solvent, preferably at room temperature, and the product may be purified by, for example, column chromatography on silica gel.

This reaction will, as in the previous reaction, generally give a mixture of products which can be separated chromatographically.

Intermediate compounds of formula (III) in which both X and Z are protected hydroxyl groups may be prepared by treating the corresponding α-hydroxycarboxylic acid (i.e. a compound of formula (III), wherein X and Z are both hydroxyl groups) with a conventional protecting agent, such as a suitable silylating agent, preferably t-butyldimethylchlorosilane or N,O-bistrimethylsilylacetamide.

Preferably, when t-butyldimethylchlorosilane is used as the silylating agent, the O-protection reaction is carried out in an anhydrous polar organic solvent, such as dimethylformamide, suitably in the presence of an amine base, preferably imidazole.

When N,O-bistrimethylsilylacetamide is the silylating agent, the reaction is preferably carried out in tetrahydrofuran.

The desired intermediate compounds of formula (III) wherein Z is a leaving group and X is a protected hydroxyl group may then be prepared by treating the di-O-protected α-hydroxycarboxylic acid with a suitable reagent for formation of the acid halide. For example, compounds of formula (III) in which X is a protected hydroxyl group and Z is a chlorine atom may be prepared by treating the bis-silylated α-hydroxycarboxylic acid with oxalyl chloride.

The deprotection of the O-protected compound of formula (I), may be carried out under acidic conditions, such as by treatment with acetic acid.

Compounds of formula (I), wherein $R^1$, $R^2$ and Y are as defined and $R^3$ is a $C_{1-6}$ alkylcarbonyl, benzoyl, or a substituted benzoyl as defined above, may be prepared by treating a 3',5'-O-substituted compound of formula (I), as defined above, with an acylating agent of formula (IV):

$$R^5.COZ \quad (IV)$$

wherein Z is as defined in formula (III) and $R^5$ is a $C_{1-6}$ alkyl group, or is a phenyl group optionally substituted with halo, nitro, $C_{1-4}$-alkyl,protected hydroxy, N-protected amino, N,N-di-$C_{1-6}$-alkylsulphamoyl, $C_{1-6}$-alkylamino, or $C_{1-4}$ alkyloxy, and optionally deprotecting the product. Suitably Z is a halogen atom, preferably chlorine.

The reaction is conveniently carried out in an anhydrous polar solvent, such as dioxane and in the presence of an amine, suitably triethylamine.

Suitable O-protecting agents, useful in the preparation of compounds of formula (I), wherein $R^3$ is a group $R^5$CO-, include silylating agents , such as N,O-bistrimethylsilylacetamide, and in which case the silylation reaction is preferably carried out in an anhydrous polar organic solvent, suitably tetrahydrofuran, at room temperature.

The removal of the O-protecting silyl groups may be performed using conventional procedures, suitably by partitioning the silylated compound between chloroform and dilute hydrochloric acid.

When the O-protecting group is a tri-$C_{1-6}$ alkylsilyl group, then deprotection of the product is an optional step, since the silylated product is itself a compound of formula (I).

Compounds of formula (I) may be converted into further compounds of formula (I). For example, in the case of compounds of formula (I), wherein $R^1$, $R^2$ and Y are as defined, X is a halogen atom, and $R^3$ is a hydrogen atom, X may be converted to a $V-\underset{\underset{O}{\|}}{C}-O-$ group wherein V is as defined in formula (I), by treatment with a carboxylic acid of formula:

$$VCO_2H$$

in the presence of a base. A preferred base is an amine, suitably a tertiary amine, such as triethylamine.

The reaction is conveniently carried out in a polar organic solvent such as dimethylformamide, and the product purified by column chromatography, on silica gel.

Further compounds of formula (I) wherein $R^3$ and Y are as defined in relation to formula (I), and $R^1$ or $R^2$ is a tri-$C_{1-6}$ alkylsilyl group and/or where the remaining substituent, $R^1$ or $R^2$, is a group RCH.(X)CO-, wherein R is as defined in relation to formula (I), and X is a tri-$C_{1-6}$ alkylsilyloxy group, may be prepared by treatment of the corresponding mono- or dihydroxy compound of formula (I) with a tri-$C_{1-6}$ alkyl silylating agent, as described above.

The compounds of formula (I) contain an additional asymmetric centre in the group(s) RCH(X)CO. A stereospecific synthesis of a compound of formula (I) may be effected by using an optically pure acylating agent of formula (III).

The compounds of formula (I) may be formulated for use in a pharmaceutical composition. Accordingly, in a further aspect of the invention, there is provided a pharmaceutical composition which comprises a compound of the formula (I) together with a pharmaceutically acceptable carrier or excipient.

Compositions which may be given by the oral route may be compounded in the form of syrups, tablets and capsules. When the composition is in the form of a tablet, any pharmaceutical carrier suitable for formulating such solid compositions may be used, for example, magnesium stearate, starch, lactose, glucose, rice, flour, and chalk. The composition may also be in the form of an ingestible capsule, for example of gelatin, to contain the compound, or in the form of a syrup, a solution, or a suspension. Suitable liquid pharmaceutical carriers include ethyl alcohol, glycerine, saline, and water to which flavouring or colouring agents may be added.

The compounds may also be presented with a sterile liquid carrier for injection. The liquid carrier suitably may be a sterile oil or sterile water.

The composition may also be formulated for topical application to the skin or eyes.

For topical application to the skin, the compounds of the invention may be made up into a cream, lotion or ointment. These formulations may be

conventional formulations well known in the art, for example, as described in standard books of pharmaceutics and cosmetics, such as Harry's Cosmeticology published by Leonard Hill Books, and the British Pharmacopaeia.

The composition for application to the eyes may be a conventional eye-drop composition well known in the art.

Preferably, the compositions of this invention are in unit dosage form or in some other form that the patient may administer to himself a single dose. A suitable dosage unit might contain from 50 mg to 1 g of active ingredient, for example 100 to 500 mg. Such doses may be administered 1 to 4 times a day or more usually 2 or 3 times a day. The effective dose of compound depends on the particular compound employed, but is in general in the range of from 1.0 to 20 mg/kg of body weight per day or more usually 2.0 to 10 mg/kg/day.

Accordingly, the present invention further provides a method for treating mammals, including humans, suffering from viral infections, which comprises administering to the sufferer a pharmaceutically effective, non-toxic, amount of a compound of formula (I).

The following Examples illustrate compounds of the invention, and the Description illustrate intermediate compounds.

Example 1

E-5-(2-Bromovinyl)-3',5'-bis(chloroacetyl)-2'-deoxyuridine

To dry dioxane (25ml) containing 1,8-diazabicyclo[5.4.0]undec-7-ene (2.7ml, 18mmol) was added chloroacetyl chloride (2.9ml, 36mmol) followed by E-5-(2-bromovinyl)-2'-deoxyuridine (2.0g, 6mmol). The solution was stirred for 0.5 hour at room temperature. Excess reagent was quenched by addition of ice and the solution was then partitioned between dilute hydrochloric acid and chloroform. The organic layer was washed with aqueous bicarbonate, dried (magnesium sulphate) and the solvent removed. The residue was purified by column chromatography on silica gel, eluting with chloroform-methanol (100:1) to give E-5-(2-bromovinyl)-3',5'-bis(chloroacetyl)-2'-deoxyuridine as a crystalline solid (2.4g, 83%), m.p. 113-115°C.
νmax (KBr) 3080 (NH), 1750,1710,1680 (C=O) $cm^{-1}$; $^1H$ nmr ($CDCl_3$) δ 2.43 (2H, m, 2'-$CH_2$), 4.17 (2H, s, $CH_2Cl$), 4.22 (2H, s, $CH_2Cl$), 4.36 (1H, m, 4'-CH), 4.53 (2H, m, 5'-$CH_2$), 5.38 (1H, m, 3'-CH), 6.33 (1H, dd, J = 6Hz and J = 9Hz, 1'-CH), 6.83 (1H, d, J = 14Hz, CH=CHBr), 7.48 (1H, d, J = 14Hz, CH=CHBr), 7.53 (1H, s, 6-CH), 9.6 (1H, s, $D_2O$ exchangeable, 3-NH).

Analysis

Found: C, 37.44; H, 3.20; N, 5.57% $C_{15}H_{15}BrCl_2N_2O_7$
requires: C, 37.06; H, 3.11; N, 5.76%.

Examples 2 and 3

E-5-(2-Bromovinyl)-5'-chloroacetyl-2'-deoxyuridine (Example 2) and
E-5-(2-Bromovinyl)-3'-chloroacetyl-2'-deoxyuridine (Example 3)

To E-5-(2-bromovinyl)-2'-deoxyuridine (3.33g, 10mmol) in dry dioxane (30ml) was added triethylamine (1.68ml, 12mmol) followed by chloroacetyl chloride (0.96ml, 12mmol) and the mixture stirred overnight at room temperature. Any excess reagent was quenched by addition of water and the mixture was partitioned between ethyl acetate and dilute hydrochloric acid. The organic layer was washed with aqueous sodium bicarbonate, dried (magnesium sulphate) and the solvent removed. The residue was purified by column chromatography on silica

gel eluting with chloroform-methanol mixtures. The first compound to elute was E-5-(2-bromovinyl)-3'-chloroacetyl-2'-deoxyuridine which was isolated as a colourless foam (80mg, 2%).
νmax (KBr) 3440, 3060, 1700, 1600 $cm^{-1}$. $^1$H nmr ($(CD_3)_2SO$) δ 2.30 (2H, m, 2'-$CH_2$), 3.66 (2H, m, 5'-$CH_2$), 4.07 (1H, m, 4'-CH), 4.43 (2H, s, $CH_2Cl$), 5.33 (2H, m, 1H $D_2O$ exchangeable, 3'-CH and 5'-OH), 6.17 (1H, t, J = 7Hz, 1'-CH), 6.83 (1H, d, J = 14Hz, CH=CHBr), 7.21 (1H, d, J = 14Hz, CH=CHBr), 8.07 (1H, s, 6-CH), 11.59 (1H, s, $D_2O$ exchangeable, 3-NH).

Analysis

Found: C, 38.82; H, 2.84; N, 6.10% $C_{13}H_{14}BrClN_2O_6$
requires: C, 38.12; H, 3.45; N, 6.84%.

The second compound to elute was E-5-(2-bromovinyl)-5'-chloroacetyl-2'-deoxyuridine which was isolated as a white crystalline solid (0.85g, 21%), m.p. 152-155°C.
νmax (KBr) 3440, 3060, 1700, 1600 $cm^{-1}$. $^1$H nmr ($(CD_3)_2SO$) δ 2.20 (2H, t, J = 6Hz, 2'-CH), 3.98 (1H, m, 4'-CH), 4.32 (3H, m, 3'-CH and 5'-$CH_2$), 4.42 (2H, s, $CH_2Cl$), 5.42 (1H, d, J = 4Hz, $D_2O$ exchangeable, 3'-OH), 6.17 (1H, t, J = 7Hz, 1'-CH), 6.92 (1H, d, J = 13Hz, CH=CHBr), 7.32 (1H, d, J = 13Hz, CH=CHBr), 7.75 (1H, s, 6-CH), 11.58 (1H, s, $D_2O$ exchangeable, 3-NH).

Analysis

Found: C, 38.25; H. 3.31; N, 6.70% $C_{13}H_{14}BrClN_2O_6$
requires: C, 38.12; H, 3.45; N, 6.84%

Examples 4 and 5

E-5-(2-Bromovinyl)-3',5'-bis(2-chloropropionyl)-2'-deoxyuridine (Example 4) and E-5-(2-Bromovinyl)-5'-(2-chloropropionyl)-2'-deoxyuridine (Example 5)

To an ice-cooled solution of E-5-(2-bromovinyl)-2'-deoxyuridine (5.0g, 15mmol) in dry pyridine (15ml) and dry dioxane (15ml) was added 2-chloropropionyl chloride (1.57ml, 18mmol) and the solution was stirred at room temperature for 1 hour. The solution was partitioned between ethyl acetate and dilute hydrochloric acid and the organic layer was washed with aqueous sodium bicarbonate, dried (magnesium sulphate) and the solvent removed. The residue was purified by column

chromatography eluting with chloroform-methanol mixtures. The first compound to elute was E-5-(2-bromovinyl)-3',5'-bis(2-chloropropionyl)-2'-deoxyuridine which was isolated as a white crystalline solid (1.9g, 25%), m.p. 158-160°C.

νmax (KBr) 3430, 3000, 1750, 1710, 1600 $cm^{-1}$. $^1H$ nmr $((CD_3)_2SO)$ δ 1.61 (3H, d, J = 7Hz, $CH_3$), 1.64 (3H, d, J = 7Hz, $CH_3$), 2.50 (2H, m, 2'-$CH_2$), 4.40 (3H, m, 4'-CH and 5'-$CH_2$), 4.77 (2H, q, J = 7Hz, 2 x ClCH(Me)CO), 5.36 (1H, m, 3'-CH), 6.19 (1H, t, J = 7Hz, 1'-CH), 7.26 (1H, d, J = 14Hz, CH=CHBr), 7.35 (1H, d, J = 14Hz, CH=CHBr), 7.84 (1H, s, 6-CH), 11.73 (1H, s, $D_2O$ exchangeable, 3-NH).

Analysis

Found: C, 39.78; H, 3.55; N, 5.22% $C_{17}H_{19}BrCl_2N_2O_7$
requires: C, 39.71; H, 3.72; N, 5.45%.

The second compound to elute was E-5-(2-bromovinyl)-5'-(2-chloropropionyl)-2'-deoxyuridine which was isolated as a white crystalline solid (2.5g, 40%), m.p. 166-173°C.

νmax (KBr) 3480, 3070, 1740, 1690, 1600 $cm^{-1}$. $^1H$ nmr $((CD_3)_2SO)$ δ 1.58 (3H, d, J = 7Hz, $CH_3$), 2.21 (2H, t, J = 6Hz, 2'-$CH_2$), 3.95 (1H, m, 4'-CH), 4.31 (3H, m, 3'-CH and 5'-$CH_2$), 4.74 (1H, q, J = 7Hz, ClCH(Me)CO), 5.43 (1H, d, J = 4Hz, $D_2O$ exchangeable, 3'-OH), 6.18 (1H, t, J = 7Hz, 1'-CH), 6.93 (1H, d, J = 13Hz, CH=CHBr), 7.33 (1H, d, J = 13Hz, CH=CHBr), 7.77 (1H, s, 6-CH), 11.65 (1H, s, $D_2O$ exchangeable, 3-NH).

Analysis

Found: C, 39.55; H, 3.65; N, 6.44%. $C_{14}H_{16}BrClN_2O_6$
requires: C, 39.69; H, 3.81; N, 6.61%.

## Example 6

### E-5-(2-Bromovinyl)-3',5'-bis(4-dipropylsulphamoylbenzoyloxyacetyl)-2'-deoxyuridine

To a solution of E-5-(2-bromovinyl)-3',5'-bis(chloroacetyl)-2'-deoxyuridine (486mg, 1.0mmol) in dimethylformamide (10ml) was added 4-dipropylsulphamoylbenzoic acid (1.42g, 5mmol) followed by triethylamine (0.70ml, 5mmol) and the solution stirred for 18 hours at room temperature. The solution was partitioned between chloroform and dilute hydrochloric acid and the organic layer was washed with aqueous sodium bicarbonate, dried (magnesium sulphate) and the solvent removed.

The residue was purified by column chromatography on silica gel, eluting with hexane-chloroform mixtures to give E-5-(2-bromovinyl)-3',5'-bis(4-dipropylsulphamoylbenzoyloxyacetyl)-2'-deoxyuridine as a colourless foam (0.79g, 77%).

λmax ($CH_3OH$) 229 (ε 29.2 x $10^3$) and 250 (ε 33.2 x $10^3$) nm.

νmax (KBr) 2960, 1770, 1740, 1600 $cm^{-1}$. $^1H$ nmr ($CDCl_3$) δ 0.98 (12H, t, J = 7Hz, 4 x $CH_3$), 1.55 (8H, sextet, J = 7Hz, 4 x $CCH_2C$), 2.30 (2H, m, 2'-$CH_2$), 3.12 (8H, t, J = 7Hz, 4 x $NCH_2$), 4.34 (1H, m, 4'-CH), 4.57 (2H, m, 5'-$CH_2$), 4.91 (2H, s, $OCH_2CO$), 4.94 (2H, s, $OCH_2CO$), 5.38 (1H, m, 3'-CH), 6.21 (1H, dd, J = 6Hz and J = 8Hz, 1'-CH), 6.72 (1H, d, J = 14Hz, CH=CHBr), 7.42 (1H, d, J = 14Hz, CH=CHBr), 7.50 (1H, s, 6-CH), 7.9-8.3 (8H, m, 2 x $C_6H_4$), 9.39 (1H, s, $D_2O$ exchangeable, 3-NH).

Analysis

Found: C, 49.87; H, 5.11; N, 5.55% $C_{41}H_{51}BrN_4O_{15}S_2$
requires: C, 50.05; H, 5.22; N, 5.69%.

## Example 7

### E-5-(2-Bromovinyl)-5'-(4-dipropylsulphamoylbenzoyloxyacetyl)-2'-deoxyuridine

To a solution of E-5-(2-bromovinyl)-5'-chloroacetyl-2'-deoxyuridine (410mg 1.0mmol) in dimethylformamide (6ml) was added 4-dipropylsulphamoylbenzoic acid (0.86g, 3mmol) followed by triethylamine (0.42ml, 3mmol) and the solution was left for 20 hours at room temperature. The solution was partitioned between chloroform and dilute hydrochloric acid and the organic layer was washed with aqueous sodium bicarbonate, dried (magnesium sulphate) and the solvent removed. The residue was purified by column chromatography on silica gel, eluting with chloroform-methanol mixtures to give E-5-(2-bromovinyl)-5'-(4-dipropylsulphamoylbenzoyloxyacetyl)-2'-deoxyuridine as a white crystalline solid (0.35g, 53%), m.p. 174-176°C.

λmax ($CH_3OH$) 250 (ε = 23.5 x $10^3$) and 285 (ε = 13.7 x $10^3$) nm.

νmax (KBr) 3500, 2980, 1730, 1690 $cm^{-1}$. $^1H$ nmr ($(CD_3)_2SO$) δ 0.82 (6H, t, J = 7Hz, 2 x $CH_3$), 1.48 (4H, sextet, J = 7Hz, 2 x $CCH_2C$), 2.20 (2H, t, J = 6Hz, 2'-$CH_2$), 3.07 (4H, t, J = 7Hz, 2 x $NCH_2$), 4.00 (1H, m, 4'-CH), 4.28 (1H, m, 3'-CH), 4.40 (2H, m, 5'-$CH_2$), 5.04 (2H, s, $OCH_2CO$),

5.5 (1H, broad, $D_2O$ exchangeable, 3'-OH), 6.21 (1H, t, J = 6Hz, 1'-CH), 6.95 (1H, d, J = 13Hz, CH=CHBr), 7.35 (1H, d, J = 13Hz, CH=CHBr), 7.81 (1H, s, 6-CH), 8.0-8.3 (4H, m, $C_6H_4$), 11.7 (1H broad, $D_2O$ exchangeable, 3-NH).

Analysis

Found: C, 47.43; H, 4.92; N, 6.24%. $C_{26}H_{32}BrN_3O_{10}S$ requires: C, 47.42; H, 4.90; N, 6.38%.

Example 8

E-5-(2-Bromovinyl)-3'-(4-dipropylsulphamoylbenzoyloxyacetyl)-2'-deoxyuridine

To a solution of E-5-(2-bromovinyl)-3'-chloroacetyl-2'-deoxyuridine (120mg, 0.3mmol) in dimethylformamide (2ml) was added 4-dipropylsulphamoylbenzoic acid (285mg, 1.0mmol) followed by triethylamine (0.14ml, 1.0mmol) and the solution was left for 18 hours at room temperature. The solution was partitioned between chloroform and water and the organic layer was washed with aqueous sodium bicarbonate, dried (magnesium sulphate) and the solvent removed. The residue was purified by column chromatography on silica gel, eluting with chloroform-methanol (100:1) to give E-5-(2-bromovinyl)-3'-(4-dipropylsulphamoylbenzoyloxyacetyl)-2'-deoxyuridine as a colourless foam (100mg, 50%).

$\nu$max (KBr) 2970, 1710, 1600 $cm^{-1}$. $^1H$ nmr ($(CD_3)_2SO$) $\delta$ 0.81 (6H, t, J = 7Hz, 2 x $CH_3$), 1.48 (4H, sextet, J = 7Hz, 2 x $CCH_2C$), 2.35 (2H, m, 2'-$CH_2$), 2.90 (4H, t, J = 7Hz, 2 x $NCH_2$), 3.64 (2H, m, 5'-$CH_2$), 4.09 (1H, m, 4'-CH), 5.04 (2H, s, $OCH_2CO$), 5.25 (1H, t, J = 5Hz, $D_2O$ exchangeable, 5'-OH), 5.35 (1H, m, 3'-CH), 6.17 (1H, t, J = 7Hz, 1'-CH), 6.83 (1H, d, J = 14Hz, CH=CHBr), 7.27 (1H, d, J = 14Hz, CH=CHBr), 8.06 (1H, s, 6-CH), 7.9-8.3 (4H, m, $C_6H_4$), 11.60 (1H, s, $D_2O$ exchangeable, 3-NH).

Analysis

Found: C, 47.34; H, 4.93; N, 6.24%. $C_{26}H_{32}BrN_3O_{10}S$ requires: C, 47.42; H, 4.90; N, 6.38%.

Example 9

E-5-(2-Bromovinyl)-3',5'-bis[2-(4-chlorophenoxy)-2-methylpropionyloxyacetyl]-2'-deoxyuridine

To a solution of E-5-(2-bromovinyl)-3',5'-bis(chloroacetyl)-2'-deoxyuridine (486mg, 1.0mmol) in dimethylformamide (5ml) was added 2-(4-chlorophenoxy)-2-methylpropionic acid (1.07g, 5mmol) followed by triethylamine (0.7ml, 5mmol) and the solution was left for 18 hours at room temperature. The solution was partitioned between chloroform and water and the organic layer was washed with aqueous sodium bicarbonate, dried (magnesium sulphate) and the solvent removed. The residue was purified by column chromatography on silica gel, eluting with chloroform-hexane mixtures to give E-5-(2-bromovinyl)-3',5'-bis[2-(4-chlorophenoxy)-2-methylpropionyloxyacetyl]-2'-deoxyuridine as a colourless foam (640mg, 76%).

$\lambda$max ($CH_3OH$) 226 ($\varepsilon = 29.0 \times 10^3$), 249 ($\varepsilon = 14.3 \times 10^3$) and 286 ($\varepsilon = 12.6 \times 10^3$) nm. $\nu$max (KBr) 3000, 1750, 1590 $cm^{-1}$. $^1H$ nmr ($CDCl_3$) $\delta$ 1.64 (12H, s, 2 x $C(CH_3)_2$), 2.30 (2H, m, 2'-$CH_2$), 4.26 (1H, m, 4'-CH), 4.48 (2H, m, 5'-$CH_2$), 4.74 (4H, s, 2 x $OCH_2CO$), 5.31 (1H, m, 3'-CH), 6.18 (1H, dd, J = 6Hz and J = 8Hz, 1'-CH), 6.73 (1H, d, J = 14Hz, CH=CHBr), 6.8-7.3 (8H, m, 2 x $C_6H_4$), 7.42 (1H, s, 6-CH), 7.47 (1H, d, J = 14Hz, CH=CHBr), 9.30 (1H, s, $D_2O$ exchangeable, 3-NH).

Analysis

Found: C, 49.64; H, 4.22; N, 3.62%. $C_{35}H_{35}BrCl_2N_2O_{13}$ requires: C, 49.90; H, 4.19; N, 3.33%.

Example 10

E-5-(2-Bromovinyl)-5'-(2-acetoxypropionyl)-2'-deoxyuridine

To a solution of E-5-(2-bromovinyl)-5'-(2-chloropropionyl)-2'-deoxyuridine (424mg, 1.0mmol) in dimethylformamide (5ml) were added potassium acetate (294mg, 3.0mmol) and 18-crown-6 (0.79g, 3mmol) and the solution was stirred for 5 days at room temperature. The solution was partitioned between ethyl acetate and water and the organic layer was then dried (magnesium sulphate) and the solvent removed. The residue was purified by column chromatography on silica gel eluting

with chloroform-methanol mixtures to give E-5-(2-bromovinyl)-5'-(2-acetoxypropionyl)-2'-deoxyuridine as a colourless foam (317mg, 71%).

νmax (KBr) 3450, 3080, 1710, 1600 $cm^{-1}$. $^1H$ nmr ($(CD_3)_2SO$) δ 1.38 (3H, d, J = 7Hz, $CHCH_3$), 2.06 (3H, s, $COCH_3$), 2.17 (2H, t, J = 6Hz, 2'-$CH_2$), 3.94 (1H, m, 4'-CH), 4.26 (3H, m, 3'-CH and 5'-$CH_2$), 4.8 (1H, broad, $D_2O$ exchangeable, 3'-OH), 5.00 (1H, q, J = 7Hz, OCH(Me)CO), 6.16 (1H, t, J = 6Hz, 1'-CH), 6.92 (1H, d, J = 13Hz, CH=CHBr), 7.32 (1H, d, J = 13Hz, CH=CHBr), 7.76 (1H, s, 6-CH), 11.66 (1H, s, $D_2O$ exchangeable, 3-NH).

Analysis

Found: C, 42.13; H, 4.25; N, 6.03%. $C_{16}H_{19}BrN_2O_8$ requires: C, 42.97; H, 4.28; N, 6.26%.

M.s. observed 446.0306 ($M^+$ theoretical 446.0322).

## Example 11

E-5-(2-Bromovinyl)-5'-(2-valeryloxypropionyl)-2'-deoxyuridine

To a solution of E-5-(2-bromovinyl)-5'-(2-chloropropionyl)-2'-deoxyuridine (424mg, 1.0mmol) in dimethylformamide (4ml) was added valeric acid (0.33ml, 3.0mmol) followed by triethylamine (0.42ml, 3.0mmol) and the solution was stirred at 60-70°C for 21 hours. The solvent was removed and the residue taken up in ethyl acetate and water. The organic layer was washed with aqueous sodium bicarbonate, dried (magnesium sulphate) and the solvent removed. The residue was purified by column chromatography on silica gel eluting with chloroform-methanol mixtures. E-5-(2-bromovinyl)-5'-(2-valeryloxypropionyl)-2'-deoxyuridine was eluted as two optical isomers. The first isomer to elute was isolated as a colourless foam (115mg, 23%).

νmax ($CHCl_3$) 3620, 3400, 2970, 1720 $cm^{-1}$. $^1H$ nmr ($(CD_3)_2SO$) δ 0.82 (3H, t, J = 6Hz, $(CH_2)_3CH_3$), 1.1-1.7 (7H, m, $CH_2CH_2CH_2CH_3$ and $OCH(CH_3)CO$), 2.23 (4H, m, $CH_2(CH_2)_2CH_3$ and 2'-$CH_2$), 3.91 (1H, m, 4'-CH), 4.25 (3H, m, 3'-CH and 5'-$CH_2$), 5.00 (1H, q, J = 7Hz, OCH(Me)CO), 5.40 (1H, d, J = 4Hz, $D_2O$ exchangeable, 3'-OH), 6.16 (1H, t, J = 6Hz, 1'-CH), 6.91 (1H, d, J = 13Hz, CH=CHBr), 7.31 (1H, d, J = 13Hz, CH=CHBr), 7.75 (1H, s, 6-CH).

Analysis

Found: C, 46.64; H, 5.03; N, 5.55%. $C_{19}H_{25}BrN_2O_8$ requires: C, 46.64; H, 5.15; N, 5.73%.

The second isomer to elute was isolated as a colourless foam (85mg, 18%). νmax ($CHCl_3$) 3620, 3400, 2970, 1720 $cm^{-1}$. $^1H$ nmr ($(CD_3)_2SO$) δ 0.83 (3H, t, J = 6Hz, $(CH_2)_3C\underline{H}_3$), 1.1-1.7 (7H, m, $CH_2C\underline{H}_2C\underline{H}_2CH_3$ and $OCH(C\underline{H}_3)CO$), 2.23 (4H, m, $C\underline{H}_2(CH_2)_2CH_3$ and 2'-$CH_2$), 3.91 (1H, m, 4'-CH), 4.25 (3H, m, 3'-CH and 5'-$CH_2$), 4.99 (1H, q, J = 7Hz, OC$\underline{H}$(Me)CO), 5.41 (1H, d, J = 4Hz, $D_2O$ exchangeable, 3'-OH), 6.17 (1H, t, J = 6Hz, 1'-CH), 6.91 (1H, d, J = 13Hz, C$\underline{H}$=CHBr), 7.32 (1H, d, J = 13Hz, CH=C$\underline{H}$Br), 7.73 (1H, s, 6-CH), 11.67 (1H, s, $D_2O$ exchangeable, 3-NH).

Analysis

Found: C, 46.30; H, 4.72; N, 5.63%. $C_{19}H_{25}BrN_2O_8$ requires: C, 46.64; H, 5.15; N, 5.73%.

## Example 12

### E-5-(2-Bromovinyl)-5'-(2-benzoyloxypropionyl)-2'-deoxyuridine

To a solution of E-5-(2-bromovinyl)-5'-(2-chloropropionyl)-2'-deoxyuridine (424mg, 1.0mmol) in dimethylformamide (4ml) was added benzoic acid (0.37g, 3.0mmol) followed by triethylamine (0.42ml, 3.0mmol) and the solution was stirred at 60-70°C for 18 hours. The solvent was removed and the residue taken up in ethyl acetate and water. The organic layer was washed with aqueous sodium bicarbonate, dried (magnesium sulphate) and the solvent removed. The residue was purified by colum chromatography on silica gel eluting with chloroform-methanol mixtures to give E-5-(2-bromovinyl)-5'-(2-benzoyloxypropionyl)-2'-deoxyuridine as a colourless foam (408mg, 80%).
λmax ($CH_3OH$) 231 ($\varepsilon = 21.2 \times 10^3$) and 290 ($\varepsilon = 10.1 \times 10^3$) nm. νmax (KBr) 3460, 3070, 1720, 1600 $cm^{-1}$. $^1H$ nmr ($(CD_3)_2SO$) δ 1.56 (3H, d, J = 7Hz, $CH_3$), 2.17 (2H, m, 2'-$CH_2$), 3.99 (1H, m, 4'-CH), 4.33 (3H, m, 3'-CH and 5'-$CH_2$), 5.1 (1H, broad, $D_2O$ exchangeable, 3'-OH), 5.28 and 5.31 (total 1H, each q, J = 7Hz, OC$\underline{H}$(Me)CO), 6.16 (1H, t, J = 7Hz, 1'-CH), 6.91 (1H, d, J = 13Hz,

CH=CHBr), 7.32 (1H, d, J = 13Hz, CH=CHBr), 7.5-8.0 (6H, m, $C_6H_5$ and 6-CH), 11.58 (1H, s, $D_2O$ exchangeable, 3-NH).

Analysis

Found: C, 48.96; H, 3.93; N, 5.31%. $C_{21}H_{21}BrN_2O_8$ requires: C, 49.52; H, 4.16; N, 5.50%.

Example 13

E-5-(2-Bromovinyl)-5'-[2-(4-dipropylsulphamoylbenzoyloxy)propionyl]-2'-deoxyuridine

To a solution of E-5-(2-bromovinyl)-5'-(2-chloropropionyl)-2'-deoxyuridine (424mg, 1.0mmol) in dimethylformamide (4ml) was added 4-dipropylsulphamoylbenzoic acid (0.85g, 3mmol) followed by triethylamine (0.42ml, 3mmol) and the the solution was stirred at 60-70°C for 19 hours. The solvent was removed and the residue taken up in ethyl acetate and water. The organic layer was twice washed with aqueous sodium bicarbonate, dried (magnesium sulphate) and the solvent removed. The residue was purified by column chromatography eluting with chloroform-methanol mixtures to give E-5-(2-bromovinyl)-5'-[2-(4-dipropylsulphamoylbenzoyloxy)propionyl]-2'-deoxyuridine as a colourless foam (524mg, 78%).

λmax ($CH_3OH$) 250 (ε = 23.1 x $10^3$) and 279 (ε = 13.9 x $10^3$) nm.

νmax ($CHCl_3$) 3620, 3400, 1760, 1720 $cm^{-1}$. $^1$H nmr ($(CD_3)_2SO$) δ 0.79 (6H, t, J = 7Hz, 2 x $CH_2CH_2CH_3$), 1.2-1.6 (7H, m, 2 x $CH_2CH_2CH_3$ and $OCH(CH_3)CO$), 2.16 (2H, m, 2'-$CH_2$), 3.04 (4H, t, J = 7Hz, 2 x $NCH_2$), 3.97 (1H, m, 4'-CH), 4.32 (3H, m, 3'-CH and 5'-$CH_2$), 4.80 (1H, broad, $D_2O$ exchangeable, 3'-OH), 5.31 and 5.34 (total 1H, each q, J = 7Hz, OCH(Me)CO), 6.16 (1H, t, J = 6Hz, 1'-CH), 6.83 (1H, d, J = 13Hz, CH=CHBr), 7.30 (1H, d, J = 13Hz, CH=CHBr), 7.76 (1H, s, 6-CH), 7.9-8.3 (4H, m, $C_6H_4$), 11.68 (1H, s, $D_2O$ exchangeable, 3-NH).

Analysis

Found: C, 48.01; H, 5.09; N, 6.16%. $C_{27}H_{34}BrN_3O_{10}S$ requires: C, 48.22; H, 5.10; N, 6.25%.

Example 14

E-5-(2-Bromovinyl)-3'-trimethylsilyl-5'-(4-dipropylsulphamoylbenzoyloxyacetyl)-2'-deoxyuridine

To a solution of E-5-(2-bromovinyl)-5'-(4-dipropylsulphamoylbenzoyloxyacetyl)-2'-deoxyuridine (0.92g, 1.4mmol) in dry dioxane (30ml) was added N,O-bis(trimethylsilyl)acetamide(1.1ml) and the solution was stirred for 0.5 hour at room temperature. The solvent was removed to give E-5-(2-bromovinyl)-3'-trimethylsilyl -5'-(4-dipropylsulphamoylbenzoyloxyacetyl)-2'-deoxyuridine as a clear oil.
$^1$H nmr ($CDCl_3$) $\delta$ 0.10 (9H, s, $Si(CH_3)_3$), 0.85 (6H, t, J = 7Hz, 2 x $CCH_3$), 1.55 (4H, sextet, J = 7Hz, 2 x $CCH_2C$), 2.20 (2H, m, 2'-CH), 3.10 (4H, t, J = 7Hz, 2 x $NCH_2$), 4.17 (2H, m, 3'-CH and 4'-CH), 4.41 (2H, m, 5'-$CH_2$), 4.88 (2H, s, $OCH_2CO$), 6.16 (1H, t, J = 7Hz, 1'-CH), 6.69 (1H, d, J = 14Hz, CH=CHBr), 7.39 (1H, d, J = 14Hz, CH=CHBr), 7.46 (1H, s, 6-CH), 7.8-8.3 (4H, m, $C_6H_4$), 9.3 (1H, broad, 3-NH).

Example 15

3-(4-Dipropylsulphamoylbenzoyl)-E-5-(2-bromovinyl)-5'-(4-dipropylsulphamoylbenzoyloxyacetyl)-2'-deoxyuridine

To a solution of E-5-(2-bromovinyl)-3'-trimethylsilyl -5'-(4-dipropylsulphamoylbenzoyloxyacetyl)-2'-deoxyuridine (0.90g, 1.25mmol) in dry dioxane (20ml), was added 4-dipropylsulphamoylbenzoyl chloride (8mmol) in dry dioxane (10ml) followed by triethylamine (1.12ml, 8mmol) and the solution stirred for 7 hours at room temperature. The solution was partitioned between chloroform and dilute hydrochloric acid and the organic layer was washed with aqueous sodium bicarbonate, dried (magnesium sulphate) and the solvent removed. The residue was purified by column chromatography on silica gel, eluting with chloroform-methanol mixtures to give 3-(4-dipropylsulphamoylbenzoyl)-E-5-(2-bromovinyl)-5'-(4-dipropylsulphamoylbenzoyloxyacetyl)-2'-deoxyuridine as a foam (80mg, 9%).

νmax ($CHCl_3$) 2970, 1760, 1730, 1710, 1670 $cm^{-1}$. $^1H$ nmr ($(CD_3)_2SO$) δ 0.80 (12H, t, J = 7Hz, 4 x $CH_3$), 1.48 (8H, sextet, J = 7Hz, 4 x $CCH_2C$), 2.31 (2H, m, 2'-$CH_2$), 3.08 (8H, t, J = 7Hz, 4 x $NCH_2$), 4.02 (1H, m, 4'-CH), 4.40 (3H, m, 3'-CH and 5'-$CH_2$), 5.04 (2H, s, $OCH_2CO$), 5.47 (1H, d, J = 4Hz, 3'-OH), 6.16 (1H, t, J = 6Hz, 1'-CH), 6.99 (1H, d, J = 13Hz, C<u>H</u>=CHBr), 7.24 (1H, d, J = 13Hz, CH=C<u>H</u>Br), 7.9-8.3 (9H, m, 2 x $C_6H_4$ and 6-CH).

Analysis

Found: C, 50.85; H, 5.22; N, 5.91%. $C_{39}H_{49}BrN_4O_{13}S_2$
requires: C, 50.59; H, 5.33; N, 6.05%.

Example 16

E-5-(2-Bromovinyl)-5'-(L-2-t-butyldimethylsilyloxy-3-phenylpropionyl)-2'-deoxyuridine

To a solution of t-butyldimethylsilyl L-2-t-butyldimethylsilyloxy-3-phenylpropionate (1.78g, 4.5mmol) in dry dichloromethane (5.5ml) containing dimethylformamide (1 drop) at $0^oC$ was added oxalyl chloride (0.45ml, 5.1mmol). The solution was stirred at toom temperature for 3 hours and the solvent was then removed. To the residue was added a solution of E-5-(2-bromovinyl)-2'-deoxyuridine (1.35, 4.0mmol) in dry tetrahydrofuran (4.5ml) and dry pyridine (4.5ml), and the solution then stirred for 45 minutes at room temperature. The solution was partitioned between chloroform and dilute hydrochloric acid and the organic layer was washed with aqueous sodium bicarbonate, dried (magnesium sulphate) and the solvent removed. The residue was purified by column chromatography on silica gel eluting with chloroform-methanol mixtures to give E-5-(2-bromovinyl)-5'-(L-2-t-butyldimethylsilyloxy-3-phenylpropionyl)-2'-deoxyuridine as a white crystalline solid (1.42g, 60%), m.p. 130-132$^oC$.

λmax ($CH_3OH$)250 (ε = 14.6 x $10^3$) and 293 (ε = 11.8 x $10^3$) nm.
$^1H$ nmr ($(CD_3)_2SO$) δ -0.26 (3H, s, $SiCH_3$), -0.13 (3H, s, $SiCH_3$), 0.72 (9H, s, $C(CH_3)_3$), 2.20 (2H, t, J = 6Hz, 2'-$CH_2$), 2.6-3.1 (2H, m, Ph<u>CH</u>$_2$), 3.96 (1H, m, 4'-CH), 4.30 (4H, m, OC<u>H</u>($CH_2$Ph)CO, 3'-CH and 5'-$CH_2$), 5.42 (1H, d, J = 4Hz, 3'-OH), 6.29 (1H, t, J = 6Hz, 1'-CH), 6.95 (1H, d, J = 13Hz, C<u>H</u>=CHBr), 7.27 (5H, s, $C_6H_5$), 7.39 (1H, d, J = 13Hz, CH=C<u>H</u>Br), 7.83 (1H, s, 6-CH), 11.68 (1H, s, $D_2O$ exchangeable, 3-NH).

Analysis

Found: C, 52.56; H, 6.01; N, 4.74%. $C_{26}H_{35}BrN_2O_7Si$
requires: C, 52.43; H, 5.92; N, 4.70%.

Example 17

E-5-(2-Bromovinyl)-5'-(L-2-hydroxy-3-phenylpropionyl)-2'-deoxyuridine

A solution of E-5-(2-bromovinyl)-5'-(L-2-t-butyldimethylsilyloxy-3-phenylpropionyl)-2'-deoxyuridine (0.89g, 1.5mmol) in 80% acetic acid (50ml) was stirred at 90°C. After 3 hours the solvent was removed. The residue was purified by column chromatography on silica gel eluting with chloroform-methanol mixtures to give E-5-(2-bromovinyl)-5'-(L-2-hydroxy-3-phenylpropionyl)-2'-deoxyuridine as a white crystalline solid (0.47g, 65%), m.p. 141-143.5°C.
λmax ($CH_3OH$) 249 (ε = 14.4 x $10^3$) and 292 (ε = 11.7 x $10^3$) nm.
νmax (KBr) 3410, 3060, 1730, 1700, 1660 $cm^{-1}$. $^1H$ nmr ($(CD_3)_2SO$) δ 2.16 (2H, t, J = 6Hz, 2'-$CH_2$), 2.6-3.1 (2H, m, Ph$CH_2$), 3.94 (1H, m, 4'-CH), 4.23 (4H, m, OCH($CH_2$Ph)CO, 3'-CH and 5'-$CH_2$), 5.38 (1H, broad, $D_2O$ exchangeable, 3'-OH), 5.64 (1H, d, J = 6Hz, $D_2O$ exchangeable PhCH$_2$CHOH),

6.19 (1H, t, J = 6Hz, 1'-CH), 6.95 (1H, d, J = 13Hz, CH=CHBr), 7.25-7.45 (6H, m, $C_6H_5$ and CH=CHBr), 7.83 (1H, s, 6-CH), 11.67 (1H, s, $D_2O$ exchangeable, 3-NH).

Analysis

Found: C, 50.01; H, 4.33; N, 5.77%. $C_{20}H_{21}BrN_2O_7$
requires: C, 49.91; H, 4.40; N, 5.82%.

Example 18

E-5-(2-Bromovinyl)-3',5'-bis(L-2-t-butyldimethylsilyloxy-3-phenylpropionyl)-2'-deoxyuridine

To a solution of t-butyldimethylsilyl L-2-t-butyldimethylsilyloxy-3-phenylpropionate (3.15g, 8.0mmol) in dry dichloromethane (10mL) containing dimethylformamide (2 drops) at 0°C was added oxalyl chloride (0.81mL. 9.2mmol). The solution was stirred at room temperature for 3 hours and the solvent was removed. To the residue was added a solution of E-5-(2-bromovinyl)-2'-deoxyuridine (1.20g, 3.6mmol) in dry tetrahydrofuran (4mL) and dry pyridine (4mL) and the resulting solution stirred for 1 hour. The solution was partitioned between chloroform and dilute hydrochloric acid and the organic layer was further extracted with aqueous sodium bicarbonate, dried ($MgSO_4$) and the solvent removed. The residue was purified by column chromatography on silica gel eluting with chloroform-hexane mixtures to give E-5-(2-bromovinyl)-3',5'-bis(L-2-t-butyldimethylsilyloxy-3-phenylpropionyl)-2'-deoxyuridine as a colourless foam (2.02g, 65%); νmax (KBr) 3390, 2960, 2940, 1760, 1720 $cm^{-1}$; $^1H$ nmr ($CDCl_3$) δ -0.15-0.00 (12H, m, 2 x $Si(CH_3)_2$), 0.85 (18H, s, 2 x $C(CH_3)_3$), 1.8-2.5 (2H, m, 2'-$CH_2$), 2.75-3.20 (4H, m, 2 x $PhCH_2$), 4.00 (1H, m, 4'-CH), 4.10-4.60 (4H, m, 2 x $OCH(CH_2Ph)CO$ and 5'-$CH_2$), 5.00 (1H, m, 3'-CH), 6.14 (1H, dd, J = 6Hz and J = 8Hz, 1'-CH), 6.73 (1H, d, J = 13Hz, CH=CHBr), 7.33 (10H, s, 2 x $C_6H_5$), 7.49 (1H, s, 6-CH), 7.53 (1H, d, J = 13Hz, CH=CHBr), 9.36 (1H, s, $D_2O$ exchangeable, 3-NH); (Found: C, 57.34; J, 6.52; N, 3.27 %; $C_{41}H_{57}BrN_2O_9Si_2$ requires C, 57.40; H, 6.70; N, 3.27%).

A small amount of 3,3',5'-tris(L-2-t-butyldimethylsilyloxy-3-phenylpropionyl)-E-5-(2-bromovinyl)-2'-deoxyuridine was also isolated as a colourless foam (340mg, 9%); νmax (KBr) 2960. 2940, 1790, 1760, 1710 $cm^{-1}$; $^1H$ nmr ($CDCl_3$) δ -0.35-0.00 (18H, m, 3 x $Si(CH_3)_2$), 0.80 (27H, s, 3 x $C(CH_3)_3$), 1.8-2.5 (2H, m, 2'-$CH_2$), 2.7-3.3 (6H, m, 3 x $PhCH_2$), 4.00 (1H, m, 4'-CH), 4.2-4.6 (4H, m, 2 x $OCH(CH_2Ph)COO$ and 5'-$CH_2$), 4.84 (1H, dd, J = 6Hz and J = 9Hz, $OCH(CH_2Ph)CON$), 5.00 (1H, m, 3'-CH), 6.10 (1H, dd, J = 6Hz and J = 8Hz, 1'-CH), 6.71 (1H, d, J = 14Hz, CH=CHBr), 7.29 (15H, s, 3 x $C_6H_5$), 7.42 (1H, s, 6-CH), 7.46 (1H, d, J = 14Hz, CH=CHBr); (Found: C, 59.20; H. 7.11; N, 2.54 %; $C_{56}H_{79}BrN_2O_{11}Si_3$ requires C, 60.03; H. 7.11; N, 2.50%).

Example 19

E-5-(2-Bromovinyl)-3',5'-bis(L-2-hydroxy-3-phenylpropionyl)-2'-deoxyuridine

To a solution of E-5-(2-bromovinyl)-3',5'-bis(L-2-t-butyldimethylsilyloxy-3-phenylpropionyl)-2'-deoxyuridine (1.20g, 1.4mmol) was added tetrabutylammonium fluoride (1M in tetrahydrofuran, 2.9mL) and the solution was stirred for 20 minutes at room temperature. The solvent was removed and the residue was purified by column chromatography on silica gel eluting with acetone-hexane mixtures to give E-5-(2-bromovinyl)-3',5'-bis(L-2-hydroxy-3-phenylpropionyl)-2'-deoxyuridine as a colourless foam (0.41g, 46%). λmax (MeOH) 249 ($\varepsilon = 13.8 \times 10^3$) and 291 ($\varepsilon = 11.3 \times 10^3$) nm; νmax (KBr) 3440, 3070, 1715, 1685, 1600 $cm^{-1}$; $^1H$ nmr [$(CD_3)_2SO$] δ 2.35 (2H, m, 2'-$CH_2$), 2.7-3.1 (4H, m, 2 x Ph$CH_2$), 4.04 (1H, m, 4'-CH), 4.1-4.4 (4H, m, 2 x OCH($CH_2$Ph)CO and 5'-$CH_2$), 5.18 (1H, m, 3'-CH), 5.74 (1H, t, J = 6Hz, $D_2O$ exchangeable, 2 x Ph$CH_2$CHOH), 6.19 (1H, t, J = 7Hz, 1'-CH), 6.99 (1H, d, J = 14Hz, CH=CHBr), 7.1-7.5 (11H, m, 2 x $C_6H_5$ and CH=CHBr), 7.91 (1H, s, 6-CH), 11.74 (1H, s, $D_2O$ exchangeable, 3-NH); (Found: C, 55.44; H, 4.26; N, 4.33 %; $C_{29}H_{29}BrN_2O_9$ requires C, 55.34; H, 4.64; N, 4.45 %).

Examples 20, 21, and 22

E-5-(2-Bromovinyl)-3',5'-bis(2-chlorophenylacetyl)-2'-deoxyuridine (22)
E-5-(2-Bromovinyl)-5'-(2-chlorophenylacetyl)-2'-deoxyuridine (21) and
E-5-(2-Bromovinyl)-3'-(2-chlorophenylacetyl)-2'-deoxyuridine (20)

To a solution of E-5-(2-bromovinyl)-2'-deoxyuridine (3.33g, 10mmol) in dry pyridine (10mL) and dry dioxane (10mL) at 0°C, 2-chlorophenylacetyl chloride (2.21mL, 14mmol) was added and the solution stirred for 1 hour at room temperature. The solution was partitioned between ethyl acetate and dilute hydrochloric acid and the organic layer was further extracted with aqueous sodium bicarbonate, dried ($MgSO_4$) and the solvent removed. The residue was purified by column chromatography on silica gel eluting with hexane-chloroform mixtures. The first compound to elute was E-5-(2-bromovinyl)-3',5'-bis(2-chlorophenylacetyl)-2'-deoxyuridine which was isolated as a colourless foam (2.03g, 32%); λmax (MeOH) 222 ($\varepsilon = 17.6 \times 10^3$), 248 ($\varepsilon = 14.4 \times 10^3$) 292 ($\varepsilon = 11.5 \times 10^3$) nm; νmax (KBr) 3440, 3070, 1755, 1710 $cm^{-1}$; $^1H$ nmr ($CDCl_3$) δ 1.7-2.6 (2H, m, 2'-$CH_2$), 4.0-4.3 (1H, m, 4'-CH), 4.45 (2H, m, 5'-$CH_2$), 5.16 (1H, m, 3'-CH), 5.33 (1H, s, PhCH(Cl)CO), 5.38 (1H, s, PhCH(Cl)CO), 6.08 (1H, dd, J = 6Hz and J = 8Hz, 1'-CH), 6.55, 6.62 (total 1H, each d, J = 14Hz, CH=CHBr), 7.2-7.5 (12H, m, 2 x $C_6H_5$, CH=CHBr and 6-CH), 9.28 (1H, s, $D_2O$ exchangeable, 3-NH); (Found: C, 51.10; H, 3.71; N, 4.31 %; $C_{27}H_{23}BrCl_2N_2O_7$ requires C, 50.87; H, 3.63; N, 4.39 %).

The second compound to elute was E-5-(2-bromovinyl)-3'-(2-chlorophenylacetyl)-2'-deoxyuridine which was isolated as a colourless foam (0.19g, 4%); νmax (KBr) 3420, 3060, 1710, 1470 $cm^{-1}$; $^1H$ nmr [$(CD_3)_2SO$] δ 2.31 (2H, m, 2'-$CH_2$), 3.63 (2H, m, 5'-$CH_2$), 3.8-4.1 (1H, m, 4'-CH), 5.12 (1H, broad, $D_2O$ exchangeable, 5'-OH), 5.35 (1H, m, 3'-CH), 5.99 (1H, s, PhCH(Cl)CO), 6.13 (1H, m, 1'-CH), 6.84 (1H, d, J = 13Hz, CH=CHBr), 7.2-7.6 (6H, m, $C_6H_5$ and CH=CHBr), 8.06 (1H, s, 6-CH), 11.59 (1H, s, $D_2O$ exchangeable, 3-NH); (Found: C, 46.92; H, 3.36; N, 5.48 %; $C_{19}H_{18}BrClN_2O_6$ requires C, 46.98; H, 3.74; N, 5.77%).

The third compound to elute was E-5-(2-bromovinyl)-5'-(2-chlorophenylacetyl)-2'-deoxyuridine which was isolated as a white crystalline solid (1.47g, 30%); m.p. 175-180°C; λmax (MeOH) 249 ($\varepsilon = 13.9 \times 10^3$) and 292 ($\varepsilon = 11.4 \times 10^3$) nm; νmax (KBr) 3380, 3070, 1760, 1700 $cm^{-1}$; $^1H$ nmr [$(CD_3)_2SO$] δ 2.1 (2H, m, 2'-$CH_2$), 3.94 (1H, m, 4'-CH), 4.1-4.4 (3H, m, 3'-CH and

5'-$CH_2$), 5.38 (1H, d, J = 4Hz, $D_2O$ exchangeable, 3'-OH), 5.93, 5.95 (total 1H, each s, PhCH(Cl)CO), 6.12 (1H, t, J = 6Hz, 1'-CH), 6.88, 6.90 (total 1H, each d, J = 14Hz, CH=CHBr), 7.2-7.5 (6H, m, $C_6H_5$ and CH=CHBr), 7.69 (1H, s, 6-CH), 11.66 (1H, s, $D_2O$ exchangeable, 3-NH); (Found: C, 46.98; H, 3.73; N, 5.73 %; $C_{19}H_{18}BrClN_2O_6$ requires C, 46.98; H, 3.74; N, 5.77 %).

Examples 23 and 24

5-(E-2-Bromovinyl)-2'-deoxy-3',5'-di-O-[(2S)-hydroxyphenylacetyl]uridine (24) and 5-(E-2-Bromovinyl)-2'-deoxy-5'-O-[(2S)-hydroxyphenylacetyl]uridine (23)

To a solution of trimethylsilyl (2S)-trimethylsilyloxyphenylacetate (3.55g, 12.0mmol) in dry dichloromethane (15ml) containing dimethylformamide (3 drops) at $0^{o}C$ was added oxalyl chloride (1.22ml, 14mmol) and the solution stirred for 1h at room temperature. The solvent was removed and to the residue at $0^{o}C$ was added a solution of 5-(E-2-bromovinyl)-2'-deoxyuridine (2.83g, 8.5mmol) in dry tetrahydrofuran (9ml) and dry pyridine (9ml) and the solution stirred for 1h at room temperature. The solution was partitioned between ethyl acetate and dilute hydrochloric acid and the organic layer was further washed with aqueous sodium bicarbonate, dried (magnesium sulphate) and the solvent removed. The residue was purified by column chromatography on silica gel eluting with chloroform -methanol mixtures. The first major product to elute was 5-(E-2-bromovinyl)-2'-deoxy-3',5'-di-O-[(2S)-hydroxyphenylacetyl]uridine which was obtained as a colourless foam (810mg, 16%); νmax (KBr) 3430, 3070, 1740 and 1710 $cm^{-1}$; $\delta_H$ [$(CD_3)_2SO$] 2.20 (2H, m, 2'-H), 4.00 (1H, m, 4'-H), 4.21 (2H, m, 5'-H), 5.0-5.2 (3H, m, 2 x PhCHOH and 3'-H), 6.0-6.2 (3H, m; 2H $D_2O$ exchangeable, 2 x OH; $D_2O$ exchange leaves 1H, t, J 6Hz, 1'-H), 6.87 (1H, d, J 14Hz, CH=CHBr), 7.2-7.5 (11H, m, 2 x $C_6H_5$ and CH=CHBr), 7.76 (1H, s, 6-H) and 11.69 (1H, s, $D_2O$ exchangeable, 3-H) (Found: C, 52.44; H, 4.16; N, 4.86 %. $C_{27}H_{25}BrN_2O_9$ requires C, 53.92; H, 4.19; N, 4.66 %).

The second major product to elute was 5-(E-2-bromovinyl)-2'-deoxy-5'-O-[(2S)-hydroxyphenylacetyl]-uridine which was obtained as a white crystalline solid (775mg, 20%), m.p. 146-150$^{o}C$; νmax (KBr) 3410, 1695, and 1470 $cm^{-1}$; $\delta_H$ [$(CD_3)_2SO$] 2.05 (2H, m, 2'-H), 3.97 (1H, m, 4'-H), 4.14 (1H, m, 3'-H), 4.26 (2H, m, 5'-H), 5.17 (1H, d, J 6Hz, collapses to s on $D_2O$ exchange, PhCHOH), 5.37

(1H, d, J 4Hz, $D_2O$ exchangeable, 3'-OH), 6.05-6.25 (2H, m, 1H $D_2O$ exchangeable, PhCHOH; $D_2O$ eachange leaves 1H, t, J 6Hz, 1'-H), 6.93 (1H, d, J 14Hz, CH=CHBr), 7.3-7.5 (6H, m, $C_6H_5$ and CH=CHBr), 7.77 (1H, s, 6-H), and 11.68 (1H, s, $D_2O$ exchangeable, 3-H) (Found: C, 48.98; H, 3.85; N, 5.75 %. $C_{19}H_{19}BrN_2O_7$ requires C, 48.84; H, 4.10; N, 6.00 %).

0097039

Example 25

5-(E-2-Bromovinyl)-2'-deoxy-5'-O-(L-2-hydroxy-4-methylpentanoyl)uridine

A solution of 5-(E-2-bromovinyl)-5'-O-(L-2-t-butyldimethylsilyloxy-4-methylpentanoyl)-2'-deoxyuridine (2.25g, 4.0mmol) in 80% acetic acid (130ml) was stirred at 90°C for 2.5h. The solvent was removed and the residue was purified by column chromatography on silica gel eluting with chloroform-methanol mixtures to afford 5-(E-2-bromovinyl)-2'-deoxy-5'-O-(L-2-hydroxy-4-methylpentanoyl)-uridine as a white crystalline solid (1.30g, 73%), m.p. 159-161°C (with decomposition); νmax (KBr) 3410, 2960, and 1690 $cm^{-1}$; $\delta_H$ [$(CD_3)_2SO$] 0.88 (6H, d, J 6Hz, $CH(CH_3)_2$), 1.3-2.0 (3H, m, $CH_2CH(CH_3)_2$), 2.21 (2H, m, 2'-H), 3.9-4.3 (5H, m, CH(OH)CO, 3'-H, 4'-H and 5'-H), 5.4 (1H. br, $D_2O$ exchangeable, OH), 5.50 (1H, d, J 6Hz, $D_2O$ exchangeable, OH), 6.23 (1H, t, J 7Hz, 1'-H), 6.98 (1H, s, J 14Hz, CH=CHBr), 7.38 (1H, d, J 14Hz, CH=CHBr), 7.87 (1H, s, 6-H), and 11.7 (1H, br, $D_2O$ exchangeable, 3-H) (Found: C, 45.74; H, 5.07; N, 6.39 %. $C_{17}H_{23}BrN_2O_7$ requires C, 45.65; H, 5.18; N, 6.26 %).

Example 26

5-(E-2-Bromovinyl)-2'-deoxy-3',5'-di-O-(L-2-hydroxy-4-methylpentanoyl)uridine

A solution of 5-(E-2-bromovinyl)-3',5'-di-O-(L-2-t-butyldimethylsilyloxy-4-methylpentanoyl)-2'-deoxyuridine (1.58g, 2.0mmol) in 80% acetic acid (130ml) was stirred at 90°C for 4 h. The solvent was removed and the residue was purified by column chromatography on silica gel eluting with chloroform-methanol mixtures. Trituration with methanol-water afforded 5-(E-2-bromovinyl)-2'-deoxy-3',5'-di-O-(L-2-hydroxy-4-methylpentanoyl)uridine as a white crystalline solid (0.65g, 58%), m.p. 87-89°C; νmax (KBr) 3440, 2960 and 1710 $cm^{-1}$; $\delta_H$ [$(CD_3)_2SO$] 0.89 (12H, d, J 6Hz, 2 x $CH(C\underline{H}_3)_2$), 1.4-2.0 (6H, m, 2 x $C\underline{H}_2C\underline{H}(CH_3)_2$), 2.4 (2H, m, 2'-H), 4.0-4.4 (5H, m, 2 x C<u>H</u>(OH)CO, 4'-H and 5'-H), 5.29 (1H, m, 3'-H), 5.42 (1H, d, J 6Hz, $D_2O$ exchangeable, OH), 5.57 (1H, d, J 6Hz, $D_2O$ exchangeable, OH), 6.24 (1H, t, J 7Hz, 1'-H), 6.96 (1H, d, J 13Hz, C<u>H</u>=CHBr), 7.37 (1H, d, J 13Hz, CH=C<u>H</u>Br), 7.93 (1H, s, 6-H), and 11.7 (1H, br, $D_2O$ exchangeable, 3-H) (Found: C, 49.24; H, 5.96; N, 4.94 %. $C_{23}H_{33}BrN_2O_9$ requires C, 49.21; H, 5.92; N, 4.99 %).

0097039

Examples 27 and 28

5-(E-2-Bromovinyl)-2'-deoxy-3',5'-di-O-(methoxyacetyl)-uridine (28) and 5-(E-2-Bromovinyl)-2'-deoxy-5'-O-(methoxyacetyl)uridine (27)

To a solution of 5-(E-2-bromovinyl)-2'-deoxyuridine (3.00g, 9.0mmol) in dry pyridine (9ml) and dry dioxan (9ml) at 0$^{o}$C was added methoxyacetyl chloride (1.15ml, 12.6mmol) and the solution was stirred for 0.5h at room temperature. The solution was partitioned between ethyl acetate and dilute hydrochloric acid and the organic layer was further washed with aqueous sodium bicarbonate, dried (magnesium sulphate) and the solvent removed. The residue was purified by column chromatography on silica gel eluting with chloroform-methanol mixtures. The first compound to elute was 5-(E-2-bromovinyl)-2'-deoxy-3',5'-di-O-(methoxyacetyl)uridine which was obtained as a white crystalline solid (1.11g, 26%), m.p 133-135$^{o}$C; $\nu$max (KBr) 3420, 3060, 1750, 1735, 1710, and 1685 cm$^{-1}$; $\delta_H$ [$(CD_3)_2SO$] 2.3-2.5 (3H, m, 2'-H and $(CD_3)_2SO$), 3.30 (6H, s, 2 x $CH_3$), 4.06 (4H, s, 2 x $OCH_2CO$), 4.30 (3H, m, 4'-H and 5'-H), 5.27 (1H, m, 3'-H), 6.12 (1H, t, J 7Hz, 1'-H), 6.85 (1H, d, J 13Hz, CH=CHBr), 7.27 (1H, d, J 13Hz, CH=CHBr), 7.79 (1H, s, 6-H), and 11.67 (1H, s, $D_2O$ exchangeable, 3-H) (Found: C, 42.80; H, 4.37; N, 6.04 %. $C_{17}H_{21}BrN_2O_9$ requires C, 42.78; H, 4.44; N, 5.87 %).

The second compound to elute was 5-(E-2-bromovinyl)-2'-deoxy-5'-O-(methoxyacetyl)uridine which was obtained as a white crystalline solid (1.64g, 45%), m.p. 140-142$^{o}$C; $\nu$max (KBr) 3420, 3230, 1750, 1715, 1680, 1670, and 1655 cm$^{-1}$; $\delta_H$ [$(CD_3)_2SO$] 2.29 (2H, t, J 6Hz, 2'-H), 3.32 (3H, s, $CH_3$), 3.96 (1H, m, 4'-H), 4.09 (2H, s, $OCH_2CO$), 4.27 (3H, m, 3'-H and 5'-H), 5.4 (1H, br, $D_2O$ exchangeable, 3'-OH), 6.17 (1H, t, J 6Hz, 1'-H), 6.92 (1H, d, J 13Hz, CH=CHBr), 7.33 (1H, d, J 13Hz, CH=CHBr), 7.78 (1H, s, 6-H), and 11.6 (1H, br, $D_2O$ exchangeable, 3-H) (Found: C, 41.54; H, 4.17; N, 7.15 %. $C_{14}H_{17}BrN_2O_7$ requires C, 41.50; H, 4.23; N, 6.91 %).

0097039

Examples 29 and 30

5-(E-2-Bromovinyl)-2'-deoxy-3',5'-di-O-[(2R)-hydroxyphenylacetyl]uridine (30) and 5-(E-2-Bromovinyl)-2'-deoxy-5'-O-[(2R)-hydroxyphenylacetyl]uridine (29)

To a solution of trimethylsilyl (2R)-trimethylsilyloxyphenylacetate (3.55g, 12.0mmol) in dry dichloromethane (15ml) containing dimethylformamide (3 drops) at $0^{o}C$ was added oxalyl chloride (1.22ml, 14mmol) and the solution stirred for 0.7h at room temperature. The solvent was removed and the residue taken up in dry tetrhydrofuran (5ml). To this solution at $0^{o}C$ was added a solution of 5-(E-2-bromovinyl)-2'-deoxyuridine (2.67g, 8.0mmol) in dry tetrahydrofuran (9ml) and dry pyridine (9ml) and the solution stirred for 0.6h at room temperature. The solution was partitioned between ethyl acetate and dilute hydrochloric acid and the organic layer was further washed with aqueous sodium bicarbonate, dried (magnesium sulphate) and the solvent removed. The residue was purified by column chromatography on silica gel eluting with chloroform-methanol mixtures. The first major product to elute was 5-(E-2-bromovinyl)-2'-deoxy-3',5'-di-O-[(2R)-hydroxyphenylacetyl]uridine which was obtained as a colourless foam (452mg, 10%); νmax (KBr) 3430, 3070, 1740, and 1710 $cm^{-1}$; $\delta_H$ [$(CD_3)_2SO$] 2.20 (2H, m, 2'-H), 4.23 (3H, m, 4'-H and 5'-H), 4.7 (br, $D_2O$ exchangeable, 2 x OH), 5.20 (3H, m, 2 x PhCHOH and 3'-H), 6.04 (1H, t, J 7Hz, 1'-H), 6.83 (1H, d, J 14Hz, CH=CHBr), 7.2-7.5 (11H, m, 2 x $C_6H_5$ and CH=CHBr), 7.71 (1H, s, 6-H), and 11.66 (1H, s, $D_2O$ exchangeable, 3-H) (Found: C, 53.59; H, 4.19; N, 4.70 %. $C_{27}H_{25}BrN_2O_9$ requires C, 53.92; H, 4.19; N, 4.66 %).

The second major product to elute was 5-(E-2-bromovinyl)-2'-deoxy-5'-O-[(2R)-hydroxyphenylacetyl]-uridine which was obtained as a colourless solid (1.03g, 28%); νmax (KBr) 3420, 2040, and 1700 $cm^{-1}$; $\delta_H$ [$(CD_3)_2SO$] 2.07 (2H, t, J 6Hz, 2'-H), 3.89 (1H, m, 4'-H), 4.22 (3H, m, 3'-H and 5'-H), 4.9 (2H, br, $D_2O$ exchangeable, PhCHOH and 3'-OH), 5.18 (1H, s, PhCHOH), 6.21 (1H, t, J 6Hz, 1'-H),

6.88 (1H, d, J 14Hz, C<u>H</u>=CHBr), 7.2-7.4 (6H, m, $C_6H_5$ and CH=C<u>H</u>Br), 7.72 (1H, s, 6-H) and 11.63 (1H, s, $D_2O$ exchangeable, 3-H) (Found: C, 48.65; H, 4.07; N, 6.17%. $C_{19}H_{19}BrN_2O_7$ requires C, 48.84; H, 4.10; N, 6.00%).

Examples 31 and 32

5-(E-2-Bromovinyl)-3',5'-di-O-(L-2-t-butyldimethylsilyloxy-4-methylpentanoyl)-2'-deoxyuridine and 5-(E-2-Bromovinyl)-5'-O-(L-2-t-butyldimethylsilyloxy-4-methylpentanoyl)-2'-deoxyurudine

To a solution of t-butyldimethylsilyl L-2-t-butyldimethylsilyloxy-4-methylpentanoate (6.12g, 17mmol) in dry dichloromethane (20ml) containing dimethylformamide (4 drops) at $0^{o}C$ was added oxalyl chloride (1.71ml, 19.6mmol). The solution was stirred for 3h at room temperature and the solvent was then removed. To the residue at $0^{o}C$ was added a solution of 5-(E-2-bromovinyl)-2'-deoxyuridine (4.00g, 12mmol) in dry pyridine (12ml) and dry tetrahydrofuran (12ml) and the solution stirred for 0.5h at room temperature. The solution was partitioned between chloroform and dilute hydrochloric acid and the organic layer was further washed with aqueous sodium bicarbonate, dried (magnesium sulphate) and the solvent removed. The residue was purified by column chromatography on silica gel eluting initially with chloroform-hexane (2:1) and then with chloroform and chloroform-methanol mixtures. The first compound to elute was 5-(E-2-bromovinyl)-3',5'-di-O-(L-2-t-butyldimethylsilyloxy-4-methylpentanoyl)-2'-deoxyuridine which was obtained as a white crystalline solid (1.90g, 20%), m.p. 89-93$^{o}C$; νmax (KBr) 2960, 2930, 2860, 1760 and 1720 $cm^{-1}$; $\delta_H$ ($CDCl_3$) 0.07 (12H, s, 2 x $Si(CH_3)_2$), 0.90 (30H, m, 2 x $C(CH_3)_3$ and 2 x $CH(CH_3)_2$), 1.4-1.9 (6H, m, 2 x $CH_2CH$-$(CH_3)_2$), 2.0-2.7 (2H, m, 2'-H), 4.2-4.5 (5H, m, 2 x CH(OSi)CO, 4'-H and 5'-H), 5.17 (1H, m, 3'-H), 6.29 (1H, dd, J 6Hz and 8Hz, 1'-H), 6.71 (1H, d, J 14Hz, CH=CHBr), 7.49 (1H, d, J 14Hz, CH=CHBr), 7.53 (1H, s, 6-H), and 9.36 (1H, s, $D_2O$ exchangeable, 3-H) (Found: C, 53.35; H, 8.02; N, 3.81 %. $C_{35}H_{61}BrN_2O_9Si_2$ requires C, 53.22; H, 7.78; N, 3.55 %).

The second compound to elute was 5-(E-2-bromovinyl)-5'-O-(L-2-t-butyldimethylsilyloxy-4-methylpentanoyl)-2'-deoxyuridine which was obtained as a white crystalline solid

(3.68g, 55%), m.p. 151-155 $^{o}$C; νmax (KBr) 3410, 2960, 2930, 2860, 1700 $cm^{-1}$; $\delta_H$ [$(CD_3)_2SO$] 0.05 (6H, s, $Si(CH_3)_2$), 0.90 (15H, m, $C(CH_3)_3$ and $CH(C\underline{H}_3)_2$), 1.4-1.9 (3H, m, $C\underline{H}_2C\underline{H}(CH_3)_2$), 2.21 (2H, t, J 6Hz, 2'-H), 3.98 (1H, m, 4'-H), 4.26 (4H, m, CH(OSi)CO, 3'-H and 5'-H), 5.27 (1H, br, $D_2O$ exchangeable, 3'-OH), 6.29 (1H, t, J 6Hz, 1'-H), 6.90 (1H, d, J 14Hz, C$\underline{H}$=CHBr), 7.33 (1H, d, J 14Hz, CH=C$\underline{H}$Br), 7.79 (1H, s, 6-H), and 11.69 (1H, s, $D_2O$ exchangeable, 3-H) (Found: C, 49.49; H, 6.56; N, 5.37 %. $C_{23}H_{37}BrN_2O_7Si$ requires C, 49.19; H, 6.64; N, 4.99 %).

Examples 33 and 34

5-(E-2-Bromovinyl)-3',5'-di-O-(2-bromobutyryl)-2'-deoxyuridine (34) and 5-(E-2-Bromovinyl)-5'-O-(2-bromobutyryl)-2'-deoxyuridine (33)

To a solution of 5-(E-2-bromovinyl)-2'-deoxyuridine (3.33g, 10.0mmol) in dry pyridine (10ml) and dry dioxan (10ml) at $0^{o}C$ was added 2-bromobutyryl bromide (2.01ml, 17.0mmol) and the solution was stirred for 0.5h at room temperature. The solution was partitioned between ethyl acetate and dilute hydrochloric acid and the organic layer was further washed with aqueous sodium bicarbonate, dried (magnesium sulphate) and the solvent removed. The residue was purified by column chromatography on silica gel eluting with chloroform-methanol mixtures. The first compound to elute was 5-(E-2-bromovinyl)-3',5'-di-O-(2-bromobutyryl)-2'-deoxyuridine which was isolated as a white crystalline solid (2.00g, 32%), m.p. 130-133$^{o}$C; $\nu$max (KBr) 3440, 2980, 1740, and 1710 $cm^{-1}$; $\delta_H$ ($CDCl_3$) 1.04, 1.07 (total 6H, each t, J 7Hz, 2 x $CH_3$), 1.9-2.7 (6H, m, 2 x $C\underline{H}_2CH_3$ and 2'-H), 4.1-4.3 (3H, m, 2 x $CH_2C\underline{H}BrCO$ and 4'-H), 4.51 (2H, m, 5'-H), 5.30 (1H, m, 3'-H), 6.26 (1H, m, 1'-H), 6.72, 6.74 (total 1H, each d, J 14Hz, C$\underline{H}$=CHBr), 7.3-7.5 (2H, m, CH=C$\underline{H}$Br and 6-H), and 9.20 (1H, s, $D_2O$ exchangeable, 3-H) (Found: C, 35.93; H, 3.52; N, 4.33 %. $C_{19}H_{23}Br_3N_2O_7$ requires C, 36.16; H, 3.67; N, 4.44 %).

The second compound to elute was 5-(E-2-bromovinyl)-5'-O-(2-bromobutyryl)-2'-deoxyuridine which was isolated as a white crystalline solid (1.33g, 28%), m.p. 160.5-163 $^{o}$C; $\nu$max (KBr) 3440, 1740, and 1700 $cm^{-1}$; $\delta_H$ [$(CD_3)_2SO$] 0.96 (3H, t, J 7Hz, $CH_3$), 1.7-2.1 (2H, m, $C\underline{H}_2CH_3$), 2.20 (2H, t, J 6Hz, 2'-H), 2.96 (1H, m, 4'-H), 4.31 (3H, m, 3'-H and 5'-H), 4.52 (1H, t, J 7Hz, $CH_2C\underline{H}BrCO$), 5.4 (1H, br, $D_2O$ exchangeable, 3'-OH), 6.15 (1H, t, J 6Hz, 1'-H), 6.90 (1H, d, J 13Hz, C$\underline{H}$=CHBr), 7.30 (1H, d, J 13Hz, CH=C$\underline{H}$Br), 7.75 (1H, s, 6-H), and 11.64 (1H, s, $D_2O$ exchangeable, 3-H) (Found: C, 37.10; H, 3.65; N, 5.65%. $C_{15}H_{18}Br_2N_2O_6$ requires C, 37.37; H, 3.76; N, 5.81 %).

0097039

Examples 35 and 36

5-(E-2-Bromovinyl)-2'-deoxy-3',5'-di-O-(phenoxyacetyl)-uridine (36) and 5-(E-2-Bromovinyl)-2'-deoxy-5'-O-(phenoxyacetyl)uridine (35)

To a solution of 5-(E-2-bromovinyl)-2'-deoxyuridine (3.33g, 10.0mmol) in dry dioxan (10ml) and dry pyridine (10ml) at $0^{o}C$ was added phenoxyacetyl chloride (2.31ml, 16.7mmol) and the solution was stirred for 0.5h at room temperature. The solution was partitioned between ethyl acetate and dilute hydrochloric acid and the organic layer was further washed with aqueous sodium bicarbonate, dried (magnesium sulphate) and the solvent removed. The residue was purified by column chromatography on silica gel eluting with chloroform-methanol mixtures. The first compound to elute was 5-(E-2-bromovinyl)-2'-deoxy-3',5'-di-O-(phenoxyacetyl)uridine which was isolated as a colourless foam (2.55g, 42%); λmax (MeOH) 217 (ε 18,900), 249 (ε 14,100), and 291 (ε 11,400) nm; νmax (KBr) 1760, 1710, 1685, and 1600 $cm^{-1}$; $\delta_H$ [$(CD_3)_2SO$] 2.37 (2H, m, 2'-H), 4.2-4.4 (3H, m, 4'-H and 5'-H), 4.77 (2H, s, $OCH_2CO$), 4.80 (2H, s, $OCH_2CO$), 5.30 (1H, m, 3'-H), 6.14 (1H, t, J 7Hz, 1'-H), 6.8-7.4 (12H, m, 2 x $C_6H_5$ and CH=CHBr), 7.80 (1H, s, 6-H), and 11.64 (1H, s, $D_2O$ exchangeable, 3-H) (Found: C, 53.98; H, 4.01; N, 4.64 %. $C_{27}H_{25}BrN_2O_9$ requires C, 53.92; H, 4.19; N, 4.66 %).

The second compound to elute was 5-(E-2-bromovinyl)-2'-deoxy-5'-O-(phenoxyacetyl)uridine which was isolated as a white crystalline solid (1.88g, 40%), m.p. 163-165$^{o}C$; λmax (MeOH) 250 (ε 14,500) and 292 (ε 11,800) nm; νmax (KBr) 3410, 1755, 1725, 1695, and 1660 $cm^{-1}$; $\delta_H$ [$(CD_3)_2SO$] 2.13 (2H, t, J 6Hz, 2'-H), 3.95 (1H, m, 4'-H), 4.29 (3H, m, 3'-H and 5'-H), 4.79 (2H, s, $OCH_2CO$), 5.4 (1H, br, $D_2O$ exchangeable, 3'-OH), 6.15 (1H, t, J 6Hz, 1'-H), 6.8-7.4 (7H, m, $C_6H_5$ and CH=CHBr), 7.76 (1H, s, 6-H), and 11.6 (1H, br, $D_2O$ exchangeable, 3-H) (Found: C, 48.73; H, 3.98; N, 5.77 %. $C_{19}H_{19}BrN_2O_7$ requires C, 48.84; H, 4.10; N, 6.00 %).

Description 1

t-Butyldimethylsilyl L-2-t-butyldimethylsilyloxy-3-phenylpropionate

To a solution of L-3-phenyllactic acid (2.50g, 16mmol) in dry dimethylformamide (8ml) was added t-butyldimethylsilyl chloride (4.75g, 31.5mmol) followed by imidazole (4.08g, 60mmol) and the solution was stirred for 6 hours at 60°C. The mixture was poured into water and extracted with hexane, and the organic layer further washed with aqueous sodium bicarbonate, dried(magnesium sulphate)and the solvent removed to afford t-butyldimethylsilyl L-2 butyldimethylsilyloxy-3-phenylpropionate as a clear colourless liquid (5.56g, 94%).

$\nu$max (film) 2930, 2860, 1740, 1720 $cm^{-1}$
$^1H$ nmr ($CDCl_3$) $\delta$ -0.21 (3H,s,$CHOSiCH_3$), -0.08 (3H,s,$CHOSiCH_3$), +0.26 (6H,s,$COOSi(CH_3)_2$), 0.82 (9H,s,$CHOSiC(CH_3)_3$), 0.95 (9H,s,$COOSiC(CH_3)_3$), 2.6-3,3 (2H,m,$PhCH_2$), 4.30 (1H,dd, J = 5Hz and J = 8Hz, $CH_2CHO$), 7.24 (5H,s,$C_6H_5$).
M.S. $[M-t-Bu]^+$ observed 337.1677 ($[M-t-Bu]^+$ theoretical 337.1655).

0097039

Description 2

t-Butyldimethylsilyl L-2-t-butyldimethylsilyloxy-4-methylpentanoate

To a solution of L-2-hydroxy-4-methylpentanoic acid (2.64g, 20mmol) in dry dimethylformamide (10ml) was added t-butyldimethylsilyl chloride (6.33g, 42mmol) followed by imidazole (5.72g, 84mmol) and the solution was stirred at 60°C for 6h. The solution was partitioned between water and hexane and the organic layer was further washed with aqueous sodium bicarbonate, dried (magnesium sulphate) and the solvent removed to afford t-butyldimethylsilyl L-2-t-butyldimethylsilyloxy-4-methylpentanoate as a clear colourless liquid (6.69g, 93%); νmax (film) 2960, 2930, 2900, 2860, 1740 and 1720 $cm^{-1}$; $\delta_H$ ($CDCl_3$) 0.01 (3H, s, CHOSi-C$\underline{H}_3$), 0.06 (3H, s, CHOSiC$\underline{H}_3$), 0.24 (6H, s, $COOSi(CH_3)_2$), 0.85-0.95 (24H, m, 2 x $C(CH_3)_3$ and CH(C$\underline{H}_3$)$_2$), 1.4-2.0 (3H, m, C$\underline{H}_2$C$\underline{H}$($CH_3$)$_2$), and 4.17 (1H, dd, J 5Hz, $CH_2$C$\underline{H}$(OSi)CO) (Found: [M-t-butyl]$^+$ 303.1830; $C_{14}H_{31}O_3Si_2$ requires 303.1809).

Description 3

Trimethylsilyl (2S)-trimethylsilylphenylacetate

To a solution of (S)-mandelic acid (2.28g, 15mmol) in dry tetrahydrofuran (15ml) was added N,O-bistrimethylsilylacetamide (7.0ml, 28mmol) and the solution was stirred at room temperature for 0.5h. The solvent was removed and the residue was placed under high vacuum at 50$^{o}$C to remove byproducts. Trimethylsilyl (2S)-trimethylsilyloxyphenylacetate was obtained as a clear pale yellow liquid (4.30g, 97%); νmax (film) 2960, 1740, and 1720 cm$^{-1}$; $\delta_H$ ($CDCl_3$) 0.21 (9H, s, $CHOSi(CH_3)_3$), 0.29 (9H, s, $COOSi(CH_3)_3$), 5.22 (1H, s, PhCH), and 7.3-7.6 (5H, m, $C_6H_5$) (Found: $[M-CH_3]^+$ 281.1008; $C_{13}H_{21}O_3Si_2$ requires 281.1029).

0097039

Description 4

Trimethylsilyl (2R)-trimethylsilyloxyphenylacetate

Trimethylsilyl (2*R*)-trimethylsilyloxyphenylacetate was prepared in an identical manner to the (2*S*) compound but substituting (*R*)-mendelic acid; νmax (film) 2960, 1740, and 1720 $cm^{-1}$; $\delta_H$ ($CDCl_3$) 0.16 (9H, s, $CHOSi(CH_3)_3$), 0.23 (9H, s, $COOSi(CH_3)_3$), 5.16 (1H, s, PhCH), and 7.3-7.6 (5H, m, $C_6H_5$) (Found: $[M-CH_3]^+$ 281.1001; $C_{13}H_{21}O_3Si_2$ requires 281.1029).

Antiviral Activity

In Vitro

Method

Vero (African Green Monkey Kidney) cells were grown to confluence in 24 well multidishes, each well being 1.6 cm in diameter. The cells were infected with Herpes Simplex type 1 virus (HFEM strain) and overlaid with 0.5 mL of 0.9% agarose (w/v) in maintenance medium in concentrations ranging from 100 to 0.03 µg/ml in half-log dilution steps, were added in 0.5 mL volume. The virus infected cultures were then incubated at 37°C for 4 days before fixing in 4% formaldehyde solution and staining with carbolfuchsin. The dishes were then examined to find what concentration of test compound caused a 50% reduction in the number of virus plaques formed ($PD_{50}$ value) and the minimum concentration of test compond which caused cytotoxicity (MTD).

Results

| Example No. | $PDD_{50}$ | | MTD (µg/mL) |
|---|---|---|---|
| | µg/ml | µM | |
| 1 | 0.14 | 0.29 | >100 |
| 2 | 0.12 | 0.29 | >100 |
| 4 | 0.14 | 0.27 | >100 |
| 5 | 0.14 | 0.33 | >100 |
| 6 | 2.03 | 2.06 | >100 |
| 7 | 0.24 | 0.37 | >100 |
| 8 | 0.22 | 0.33 | >100 |
| 9 | 1.47 | 1.75 | >100 |
| 10 | 0.18 | 0.40 | >100 |
| 14 | 0.32 | 0.48 | >100 |
| 16 | 0.63 | 0.68 | >100 |
| 18 | >100 | – | >100 |
| 19 | 0.43 | 0.68 | >100 |
| 20 | 0.18 | 0.37 | >100 |
| 21 | 0.07 | 0.14 | >100 |
| 22 | 0.34 | 0.53 | >100 |
| 23 | 0.16 | 0.34 | >100 |
| 24 | 0.45 | 0.75 | >100 |
| 25 | 0.15 | 0.34 | >100 |
| 26 | 0.16 | 0.29 | >100 |
| 27 | 0.12 | 0.30 | >100 |
| 28 | 0.17 | 0.36 | >100 |
| 29 | 0.22 | 0.47 | >100 |
| 30 | 0.66 | 1.10 | >100 |
| 31 | 0.53 | 0.94 | >100 |
| 32 | 4.9 | 6.2 | >100 |
| 33 | 0.16 | 0.37 | >100 |
| 34 | 0.20 | 0.32 | 100 |
| 35 | 0.18 | 0.38 | >100 |
| 36 | 0.57 | 0.95 | >100 |

0097039

CLAIMS

1. A compound of formula (I):

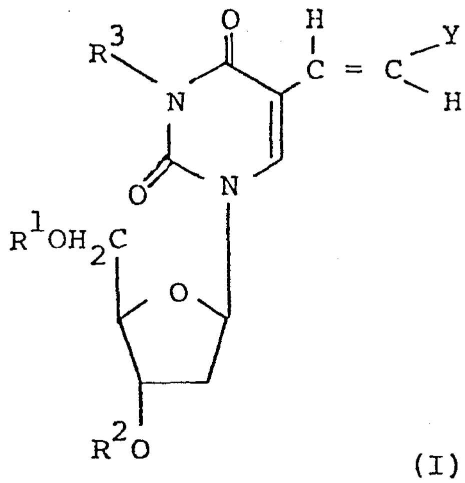

(I)

in which one of $R^1$ or $R^2$ is a group of formula $RCH(X)CO.-$, and the other is selected from: a hydrogen atom, a tri-$C_{1-6}$ alkylsilyl group, and a group of formula:

$$RCH(X)CO.-$$

wherein X is a halogen atom, a $C_{1-6}$ alkyloxy group, a phenoxy group, a hydroxyl group, a tri-$C_{1-6}$ alkylsilyloxy group, or a group of formula:

$$V - \underset{\underset{O}{\|}}{C} - O-$$

wherein V is a $C_{1-6}$ alkyl group optionally substituted with halophenoxy, or is a phenyl group optionally substituted with halogen, nitro, $C_{1-4}$-alkyl, hydroxy, amino, $C_{1-6}$-alkylamino, N,N-di-$C_{1-6}$ alkylsuphamoyl, or $C_{1-4}$ alkyloxy, and R is a hydrogen atom, a $C_{1-6}$ alkyl group, a phenyl group, a benzyl group, or a phenyl or

benzyl group substituted in the aromatic ring with halo, nitro, $C_{1-4}$-alkyl, hydroxy, amino, $C_{1-6}$-alkylamino, or $C_{1-4}$ alkyloxy; $R^3$ is a hydrogen atom, a $C_{1-6}$ alkanoyl group, a benzoyl group or a benzoyl group substituted with halo, nitro, $C_{1-4}$ alkyl, hydroxy, amino, N,N-di-$C_{1-6}$-alkylsulphamoyl, $C_{1-6}$ alkylamino, or $C_{1-4}$ alkyloxy; and Y is a halogen atom,

2. A compound according to claim 1, wherein Y is a bromine atom.

3. A compound according to claim 1 or 2, wherein X is a chlorine atom or is a hydroxy group.

4. A compound according to claim 1 or 2, wherein X is a $C_{1-6}$ alkoxy group.

5. A compound according to claim 1 or 2, wherein X is a group VCO.O- as defined in claim 1.

6. A compound according to any one of claims 1 to 5, wherein $R^3$ is a hydrogen atom.

7. A compound according to claim 1, selected from:

E-5-(2-bromovinyl)-3'-trimethylsilyl-5'-(4-dipropyl-sulphamoylbenzoyloxyacetyl)-2'-deoxyuridine;
3-(4-dipropylsulphamoylbenzoyl)-E-5-(2-bromovinyl)-5'-(4-dipropylsulphamoylbenzoyloxyacetyl)-2'-deoxyuridine;
E-5-(2-bromovinyl)-5'-(L-2-t-butyldimethylsilyloxy-3-phenylpropionyl)-2'-deoxyuridine;
E-5-(2-bromovinyl)-3',5'-bis(L-2-t-butyldimethyl-silyloxy-3-phenylpropionyl)-2'-deoxyuridine;

E-5-(2-bromovinyl)-5'-(L-2-hydroxy-3-phenylpropionyl)-2'-deoxyuridine;

E-5-(2-bromovinyl)-3',5'-bis(L-2-hydroxy-3-phenylpropionyl)-2'-deoxyuridine;

E-5-(2-bromovinyl)-3'-(2-chlorophenylacetyl)-2'-deoxyuridine;

E-5-(2-bromovinyl)-5'-(2-chlorophenylacetyl)-2'-deoxyuridine;

E-5-(2-bromovinyl)-3',5'-bis(2-chlorophenylacetyl)-2'-deoxyuridine;

E-5-(2-bromovinyl)-5'-(2-L-hydroxyphenylacetyl)-2'-deoxyuridine;

E-5-(2-bromovinyl)-3',5'-bis(2-L-hydroxyphenylacetyl)-2'-deoxyuridine;

E-5-(2-bromovinyl)-5'-(L-2-hydroxy-4-methylpentanoyl)-2'-deoxyuridine;

E-5-(2-bromovinyl)-3',5'-bis(L-2-hydroxy-4-methylpentanoyl)-2'-deoxyuridine;

E-5-(2-bromovinyl)-5'-methoxyacetyl-2'-deoxyuridine;

E-5-(2-bromovinyl)-3',5'-bis(methoxyacetyl)-2'-deoxyuridine;

E-5-(2-bromovinyl)-5'-(2-D-hydroxyphenylacetyl)-2'-deoxyuridine;

E-5-(2-bromovinyl)-3',5'-bis(2-D-hydroxyphenylacetyl)-2'-deoxyuridine;

E-5-(2-bromovinyl)-5'-(L-2-t-butyldimethylsilyloxy-4-methylpentanoyl)-2'-deoxyuridine;

E-5-(2-bromovinyl)-3',5'-bis(L-2-t-butyldimethylsilyloxy-4-methylpentanoyl)-2'-deoxyuridine;

E-5-(2-bromovinyl)-3',5'-bis(chloroacetyl)-2'-deoxyuridine;

E-5-(2-bromovinyl)-5'-chloroacetyl-2'-deoxyuridine;

E-5-(2-bromovinyl)-3'-chloroacetyl-2'-deoxyuridine;

E-5-(2-bromovinyl)-3',5'-bis(2-chloropropionyl)-2'-deoxyuridine;

E-5-(2-bromovinyl)-5'-(2-chloropropionyl)-2'-deoxyuridine;
E-5-(2-bromovinyl)-3',5'-bis(4-dipropylsulphamoylbenzoyloxyacetyl)-2'-deoxyuridine;
E-5-(2-bromovinyl)-5'-(4-dipropylsulphamoylbenzoyloxyacetyl)-2'-deoxyuridine;
E-5-(2-bromovinyl)-3'-(4-dipropylsulphamoylbenzoyloxyacetyl)-2'-deoxyuridine;
E-5-(2-bromovinyl)-3',5'-bis[2-(4-chlorophenoxy)-2-methylpropionyloxyacetyl]-2'-deoxyuridine;
E-5-(2-bromovinyl)-5'-(2-acetoxypropionyl)-2'-deoxyuridine;
E-5-(2-bromovinyl)-5'-(2-valeryloxypropionyl)-2'-deoxyuridine;
E-5-(2-bromovinyl)-5'-(2-benzoyloxypropionyl)-2'-deoxyuridine;
E-5-(2-bromovinyl)-5'-[2-(4-dipropylsulphamoylbenzoyloxy)propionyl]-2'-deoxyuridine;
E-5-(2-bromovinyl)-5'-(2-bromobutyryl)-2'-deoxyuridine;
E-5-(2-bromovinyl)-3',5'-bis(2-bromobutyryl))-2'-deoxyuridine;
E-5-(2-bromovinyl)-5'-phenoxyacetyl-2'-deoxyuridine; and
E-5-(2-bromovinyl)-3',5'-bis(phenoxyacetyl)-2'-deoxyuridine.

8. A process for preparing a compound as claimed in claim 1, which comprises treating a compound of formula (II):

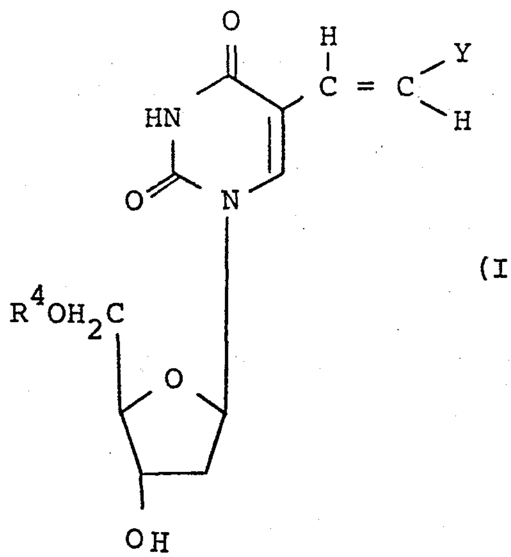

(II)

in which Y is as defined in formula (1), claim 1, $R^4$ is a hydrogen atom, or a tri-$C_{1-6}$-alkylsilyl group, with an acylating agent of formula (III):

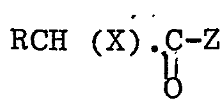

(III)

in which Z is a good leaving group, and R and X are as defined in claim 1, provided that when X is a hydroxyl group it is a protected hydroxyl group, and optionally thereafter deprotecting the product.

9. A pharmaceutical composition comprising a compound according to any one of claims 1 to 7 in combination with a pharmaceutically acceptable carrier.

10. A compound according to any one of claims 1 to 7, or a composition according to claim 9, for use in the treatment of viral infections.

0097039

European Patent Office

# EUROPEAN SEARCH REPORT

Application number

EP 83 30 3361

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X,Y | DE-A-3 010 399 (K. GAURI)<br>* Pages 5-7 *<br>--- | 1-10 | C 07 H 19/08<br>A 61 K 31/70 |
| Y | DE-A-2 915 254 (UNIVERSITY OF BIRMINGHAM)<br>* Pages 1-2 *<br>--- | 1-10 | |
| D,P Y | EP-A-0 061 283 (BEECHAM)<br>* Pages 3-4 *<br>----- | 1-10 | |
| | | | TECHNICAL FIELDS SEARCHED (Int. Cl. 3) |
| | | | C 07 H 19/00<br>A 61 K 31/00 |

The present search report has been drawn up for all claims

| Place of search | Date of completion of the search | Examiner |
|---|---|---|
| THE HAGUE | 26-08-1983 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82